(19) Europäisches Patentamt
European Patent Office
Office européen des brevets

(11) Numéro de publication: **0 443 957 B1**

(12) **FASCICULE DE BREVET EUROPEEN**

(45) Date de publication de fascicule du brevet: **10.05.95**

(51) Int. Cl.[6]: **C07J 53/00**, A61K 31/56, //C07J1/00

(21) Numéro de dépôt: **91400473.4**

(22) Date de dépôt: **21.02.91**

Le dossier contient des informations techniques présentées postérieurement au dépôt de la demande et ne figurant pas dans le présent fascicule.

(54) **Nouveaux produits stéroides comportant un radical spiro en position 17, leur procédé de préparation et les intermédiaires de ce procédé, leur application à titre de médicaments et les compositions pharmaceutiques les renfermant.**

(30) Priorité: **22.02.90 FR 9002177**

(43) Date de publication de la demande: **28.08.91 Bulletin 91/35**

(45) Mention de la délivrance du brevet: **10.05.95 Bulletin 95/19**

(84) Etats contractants désignés: **AT BE CH DE DK ES FR GB GR IT LI LU NL SE**

(56) Documents cités:
**FR-A- 2 077 877**
**US-A- 3 968 132**

(73) Titulaire: **ROUSSEL-UCLAF**
**35, Boulevard des Invalides**
**F-75007 Paris (FR)**

(72) Inventeur: **Gaillard-Kelly, Martine**
**54, rue Notre Dame de Lorette**
**F-75009 Paris (FR)**
Inventeur: **Nedelec, Lucien**
**45, Boulevard de l'Ouest**
**F-93340 Le Raincy (FR)**
Inventeur: **Nique, François**
**7, Allée Pierre Brossolette**
**F-93320 Pavillons Sous Bois (FR)**
Inventeur: **Philibert, Daniel**
**16, rue Chevalier**
**F-94210 La Varenne Saint Hilaire (FR)**

(74) Mandataire: **Bourgouin, André et al**
**Département des Brevets**
**ROUSSEL UCLAF**
**111, route de Noisy**
**B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux produits stéroïdes comportant un radical spiro en position 17, leur procédé de préparation et les intermédiaires de ce procédé, leur application comme médicaments et les compositions pharmaceutiques les renfermant.

Dans le brevet français FR 2.077.877 est décrit le 3,20 dioxo-17α,21-diméthyl-19-nor-pregna-4,9-diène. Ce produit diffère des produits de la présente demande en ce qu'il ne comporte pas de liaison entre le groupe 17α méthyle et le groupe 21 méthyle (cette liaison forme le groupe cyclopentyle condensé en position 17 des composés de la demande).

L'invention a pour objet, les produits de formule générale (I) :

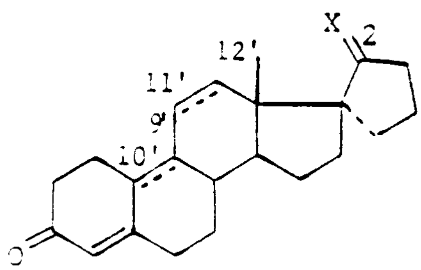

(I)

dans laquelle =X représente une fonction cétonique ou un radical

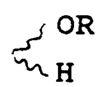

dans lequel R représente un atome d'hydrogène ou un radical acyle ayant au plus 12 atomes de carbone et les traits ondulés signifient que les substituants OR et hydrogène peuvent se trouver en positions alpha ou béta, les traits pointillés en positions 9'(10') et 11'(12') indiquent la présence éventuelle d'une seconde double liaison en position 9'(10') ou de deux double liaisons supplémentaires en positions 9'(10') et 11'(12') sous forme de chacun des épimères 2R ou 2S ou sous forme d'un mélange de ces épimères, le trait pointillé en position 17 indique que la liaison est en position alpha.

Parmi les valeurs acyle que peut représenter R on peut citer les radicaux acyle dérivés des acides carboxyliques. On préfère les radicaux acétyle, propionyle ou benzoyle.

Du fait de la présence d'un carbone asymétrique sur le cycle spiro en position 2 lorsque X représente un radical

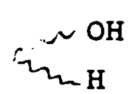

deux épimères peuvent exister. L'invention concerne bien entendu chacun de ces épimères (2R ou 2S) ainsi que leur mélange éventuel.

Les produits peuvent se présenter sous forme de produits 3'-céto delta4' lorsque les deux traits pointillés en positions 9'(10') et 11'(12') ne représentent pas la présence d'une double liaison entre les carbones qui les portent. L'invention concerne également les produits 3'-céto delta4',9'(10') et les triénones 3'-céto delta4',9'(10'), 11'(12').

Parmi les produits de formule (I) on préfère les produits dans lesquels X représente un atome d'oxygène et le trait pointillé en position 11'(12') n'indique pas la présence d'une seconde liaison entre les carbones qui le portent.

Parmi les produits de formule (I), on préfère les produits décrits ci-après dans les exemples et particulièrement le spiro (cyclopentane-1,17'béta-estra-4,9-diène) 2,3'-dione.

L'invention a également pour objet un procédé de préparation des produits de formule générale (I) telle que définie ci-dessus, caractérisé en ce que l'on soumet un produit de formule (II) :

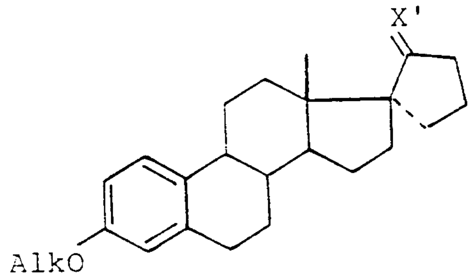

(II)

dans laquelle =X' représente une fonction cétonique éventuellement protégée ou un radical

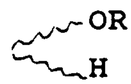

R ayant la définition indiquée ci-dessus,
sous forme de chacun des épimères 2R ou 2S ou sous forme d'un mélange de ces épimères et Alk représente un radical alkyle ayant de 1 à 4 atomes de carbone
soit à une réduction suivie d'une hydrolyse pour obtenir les produits de formule ($I_a$) :

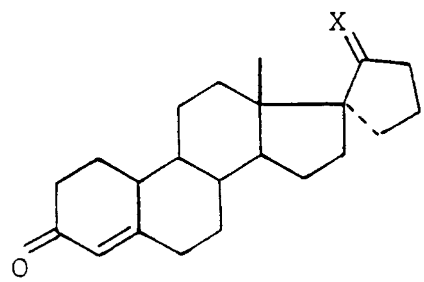

($I_a$)

dans laquelle X a la signification indiquée ci-dessus, et correspondant aux produits de formule (I) dans laquelle les traits pointillés en position 9'(10') et 11'(12') ne représentent pas une seconde liaison entre les carbones qui les portent,
soit à une réduction suivie d'une hydrolyse douce puis d'une déshydrogénation pour obtenir les produits de formule ($I_b$) :

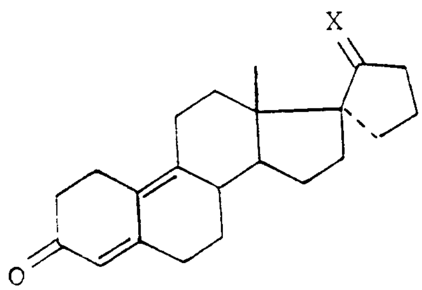

($I_b$)

correspondant aux produits de formule (I) dans laquelle les traits pointillés en positions 9'(10') indiquent la présence d'une double liaison et ceux en position 11'(12') ne représentent pas de double liaison et produits de formule ($I_b$) que l'on soumet éventuellement à une réaction de protection en position 3, suivie d'une hydrolyse douce puis d'une déshydrogénation pour obtenir les produits de formule ($I_c$) :

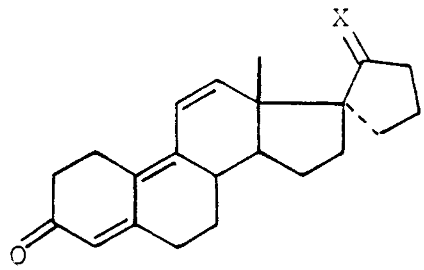

(I$_c$)

correspondant aux produits de formule (I) dans laquelle les traits pointillés en positions 9'(10') et 11'(12') indiquent la présence de deux double liaisons supplémentaires en position 9'(10') et 11'(12'),
et si désiré soumet les produits de formules ($I_a$), ($I_b$) et ($I_c$) indiquées ci-dessus et dans lesquelles =X représente un radical

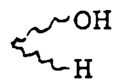

soit à une réaction d'oxydation pour obtenir les produits de formules ($I_a$), ($I_b$) et ($I_c$) correspondants dans lesquels X représente un atome d'oxygène,
soit à une réaction d'acylation pour obtenir les produits dans lesquels =X représente un radical

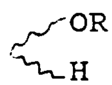

dans lequel R représente un radical acyle.

Lorsque le radical X' représente une fonction cétonique protégée, il s'agit de préférence d'une fonction cétal cyclique ou non-cyclique. On peut citer par exemple les fonctions diméthyl et diéthylcétal ou la fonction éthylène dioxy.

Parmi les valeurs alkyle que peut représenter alk dans le produit de formule (II), on peut citer méthyle, éthyle, propyle linéaire ou ramifié, butyle linéaire ou ramifié. On préfère la valeur méthyle.

La réduction à laquelle on soumet d'abord les produits de formule (II) pour obtenir les produits de formule ($I_a$) est de préférence une réaction dite de BIRCH effectuée à l'aide de lithium dans l'ammoniac liquide en présence d'un alcool qui peut être par exemple le méthanol, l'éthanol ou le tert-butanol.

L'hydrolyse que l'on effectue ensuite est de préférence opérée à l'aide d'un acide fort tel que l'acide chlorhydrique, l'acide paratoluène sulfonique ou l'acide oxalique. On opère dans un solvant tel que le méthanol. Cette hydrolyse permet normalement la transformation en cétone du radical X' lorsque celui-ci représente un fonction cétonique protégée.

La préparation des produits de formule ($I_b$) est réalisée d'abord à l'aide d'une réduction de BIRCH telle qu'énoncée ci-dessus. L'hydrolyse douce est effectuée ensuite à l'aide d'un acide faible tel que l'acide acétique ou propionique.

La déshydrogénation finale est effectuée de préférence selon la méthode dite bromation débromhydratation. La bromation est effectuée de préférence à l'aide de perbromure de pyridinium. La débromhydratation est effectuée à l'aide d'une base telle que la pyridine ou la triéthylamine. Ces deux réactions peuvent être réalisées simultanément en utilisant le mélange perbromure de pyridinium-pyridine.

Lors de la transformation éventuelle des produits de formule ($I_b$) en produits de formule ($I_c$), la protection de la fonction cétone en position 3 est effectuée comme indiqué précédemment pour X'. L'hydrolyse douce que l'on effectue ensuite est réalisée tel qu'indiqué ci-dessus.

La déshydrogénation finale est effectuée de préférence à l'aide de chloranile ou de dichlorodicyanobenzoquinone (DDQ).

L'oxydation éventuelle des produits de formule ($I_a$), ($I_b$) et ($I_c$) dans lesquelles =X représente un radical

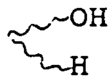

est effectuée de préférence à l'aide de réactifs usuels tels que l'acide chromique dans l'acétone (réactif de Jones), l'isopropylate d'aluminium en présence de cyclohexanone (réactif d'Oppenauer) ou le chlorochromate de pyridinium.

Lorsque, dans les produits de formule (II), ($I_a$), ($I_b$) et ($I_c$), =X' ou =X représentent un radical

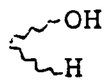

le mélange des épimères peut être factionné en chacun de ses épimères. On utilise pour cette séparation des méthodes usuelles telles que la chromatographie sur colonne de silice.

L'estérification éventuelle de l'alcool est effectuée de préférence à l'aide d'un dérivé réactif du radical acyle tel qu'un halogénure d'acide, de préférence le chlorure d'acide, un anhydride mixte ou symétrique. On peut également opérer avec l'acide carboxylique correspondant au radical acyle que l'on veut introduire en utilisant un agent de déshydratation tel que le dicyclohexyle carbodiimide (DCC).

L'invention a également pour objet un procédé de préparation des produits de formule (I') correspondant aux produits de formule générale (I) telle que décrite ci-dessus, dans laquelle =X représente un radical

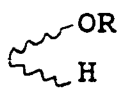

R ayant la signification indiquée précédemment, caractérisé en ce que l'on soumet un produit de formule (II') :

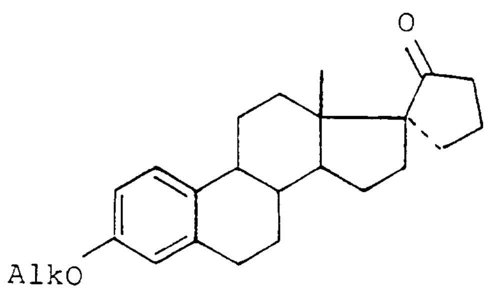

(II')

à une réaction de réduction pour obtenir les produits de formule (III) :

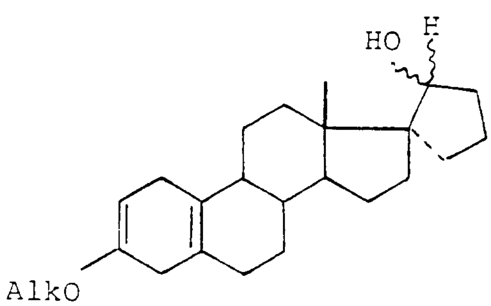

(III)

produits de formule (III) que l'on sépare si désiré en chacun de leurs isomères et soumet les produits de formule (III) sous forme de mélange d'épimères ou sous forme de produits purs, soit à une hydrolyse pour obtenir les produits de formule (I'$_a$) :

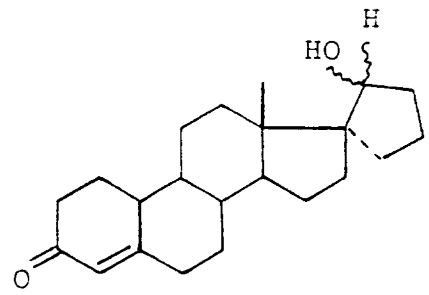

(I'$_a$)

produits de formule (I'$_a$) que l'on sépare si désiré en chacun de leurs isomères,
soit à une hydrolyse douce suivie d'une déshydrogénation pour obtenir les produits de formule (I'$_b$) :

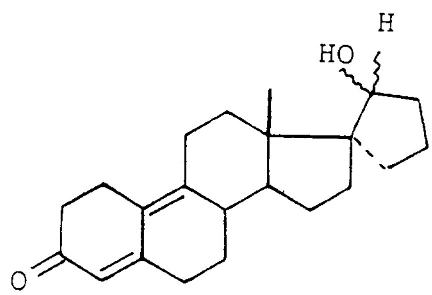

(I'$_b$)

produits de formule (I'$_b$) que l'on sépare si désiré en chacun de leurs isomères et produits de formule (I'$_b$) sous forme de mélange d'épimères ou sous forme de produits purs que l'on soumet éventuellement à une réaction de protection en position 3 suivie d'une hydrolyse douce puis d'une déshydrogénation pour obtenir les produits de formule (I'$_c$) :

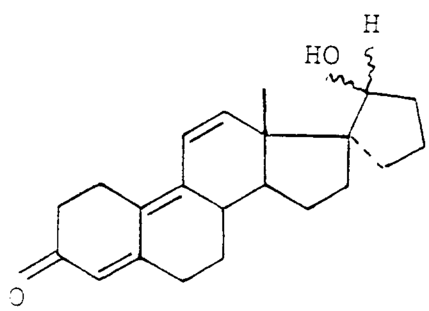

(I'$_c$)

produits de formule (I'$_c$) que l'on sépare si désiré en chacun de leurs isomères et soumet si désiré les produits de formule (I'$_a$), (I'$_b$) et (I'$_c$) ou le cas échéant leurs isomères à une réaction d'acylation pour obtenir les produits correspondants dans lesquels X représente un radical

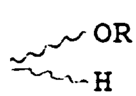

dans lequel R représente un radical acyle.

La réaction de réduction à laquelle on soumet les produits de formule (II') pour obtenir les produits de formule (III) est de préférence une réaction de BIRCH effectuée dans les conditions indiquées ci-dessus.

L'hydrolyse à laquelle on soumet les produits de formule (III) pour obtenir les produits de formule ($I'_a$) est une hydrolyse effectuée de préférence avec un acide fort tel que ceux indiqués ci-dessus.

L'hydrolyse douce puis la déshydrogénation effectuées sur les mêmes produits de formule (III) pour obtenir les produits de formule ($I'_b$) sont d'une part une hydrolyse à l'aide d'un acide faible et d'autre part une bromation débromhydratation telles que celles indiquées ci-dessus pour la préparation des produits de formule ($I_b$).

La protection en position 3 puis l'hydrolyse douce et enfin la déshydrogénation des produits de formule ($I'_b$) pour arriver aux produits de formule ($I'_c$) sont effectuées dans les mêmes conditions que celles indiquées ci-dessus pour la transformation des produits de formule ($I_b$) en produits de formule ($I_c$).

La séparation éventuelle des mélanges d'isomères est effectuée selon les méthodes usuelles telles que la chromatographie.

Lors de la transformation des produits de formule ($I_b$) en produits de formule ($I_c$), on obtient intermédiairement les produits de formule (IV) :

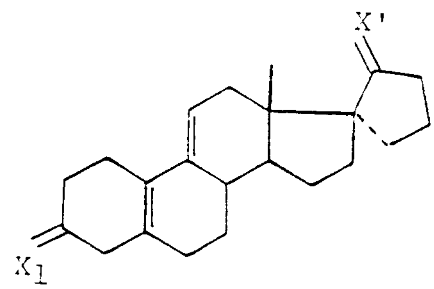

(IV)

dans laquelle X' a la signification indiquée ci-dessus et $X_1$ représente une fonction cétonique protégée.

Les produits de formule (I) présentent d'intéressantes propriétés pharmacologiques, notamment, une activité progestomimétique et une activité anti-estrogène remarquables, comme le montrent les résultats de tests exposés ci-après dans la partie expérimentale. Ces propriétés rendent lesdits produits de formule (I) aptes à être utilisés comme médicaments progestatifs inhibiteurs hypophysaires à prédominance anti-LH ou comme anti-estrogènes. Ils trouvent leur emploi dans le traitement des dysménorrhées, des stérilités, des dystrophies ovariennes par mise au repos des ovaires, dans le traitement des tumeurs du sein et de l'utérus, et comme contraceptifs. Les produits de formule (I) peuvent également être utilisés dans le traitement hormonal de la ménopause et de l'ostéoporose.

L'invention a donc pour objet lesdits produits de formule (I) à titre de médicaments.

L'invention a spécialement pour objet, à titre de médicament, les composés de formule (I) pour lesquels X représente un atome d'oxygène et le trait pointillé en position 11'(12') n'indique pas la présence d'une seconde liaison entre les carbones qui les portent.

L'invention a plus spécialement pour objet, à titre de médicaments, les produits décrits ci-après dans les exemples et en particulier le spiro (cyclopentane-1,17'béta-estra-4',9'-diène) 2,3'-dione.

La posologie utile varie en fonction de l'affection à traiter et de la voie d'administration ; elle peut aller, par exemple de 100 microgrammes à 10 mg par jour, par voie orale, chez la femme adulte, pour le produit de l'exemple 2. Lesdits produits de formule (I) sont utilisés par voie buccale, rectale, parentérale ou par voie locale en application topique sur la peau et les muqueuses. Ils peuvent être prescrits sous forme de comprimés, de comprimés enrobés, de cachets, de capsules, de granulés, d'émulsions, de sirops, de suppositoires, de solutés et de suspensions injectables, de pommades, de crèmes, de gels, de préparations en aérosols et de patchs.

L'invention a donc également pour objet les compositions pharmaceutiques renfermant, comme principe actif, au moins un composé de formule (I).

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs. Ces compositions pharmaceutiques peuvent être préparées selon les méthodes usuelles.

Les produits de formule (II) sont des produits nouveaux. Ils peuvent être préparés comme suit :

On fait agir du lithium dans l'ammoniac liquide sur un produit de formule (V) :

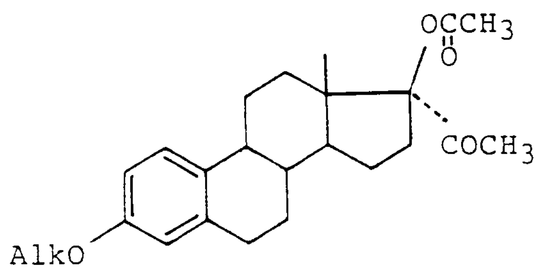

(V)

dans laquelle Alk a la signification précédente, puis ajoute un produit de formule (VI) :

$Hal\text{-}CH_2\text{-}CH=CH_2$ (VI)

dans laquelle Hal représente un atome d'halogène pour obtenir les produits de formule (VII) :

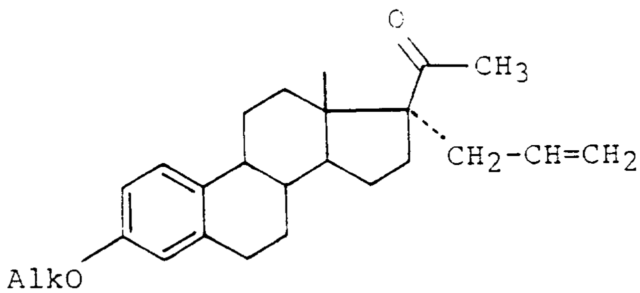

(VII)

produits de formule (VII) que l'on soumet à une ozonolyse pour obtenir les produits de formule (VIII) :

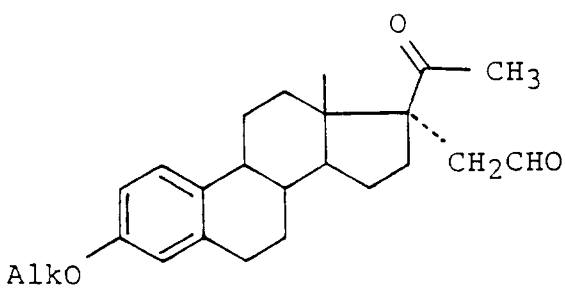

(VIII)

que l'on traite par une base pour obtenir les produits de formule (IX) :

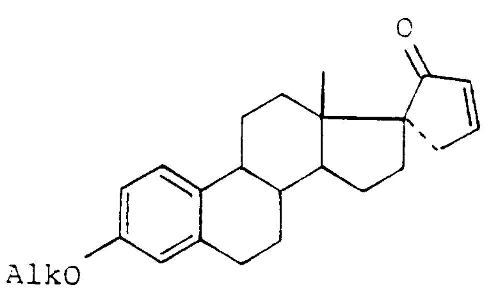

(IX)

que l'on soumet à une hydrogénation pour obtenir les produits de formule (X) :

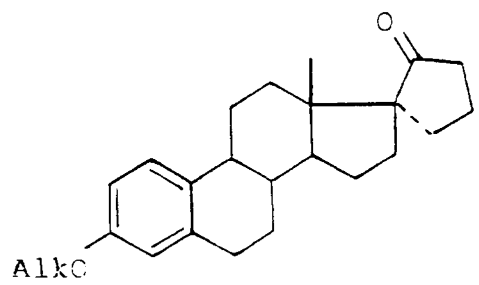

(X)

produits de formule (X) que :
soit l'on soumet à une réaction de protection de la fonction cétone pour obtenir les produits de formule (II) dans lesquels X' représente une cétone protégée,
soit l'on soumet à une réaction de réduction suivie éventuellement d'une séparation des isomères pour obtenir les produits de formule (II) dans lesquels =X' représente un radical

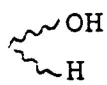

Lesdits produits peuvent être soumis à une réaction d'acylation pour obtenir les produits de formule (II) dans laquelle =X' représente le radical

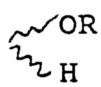

dans lequel R représente un radical acyle.

Dans la préparation indiquée ci-dessus, l'atome d'halogène que représente Hal est de préférence un atome de brome. La base avec laquelle on traite les produits de formule (VIII) pour obtenir les produits de formule (IX) est de préférence la potasse dans le méthanol.

L'hydrogénation à laquelle on soumet les produits de formule (IX) pour obtenir les produits de formule (X) est de préférence effectuée par l'hydrogène en présence d'un catalyseur tel que le palladium sur charbon actif. On opère de préférence dans un solvant tel que le tétrahydrofuranne.

La protection éventuelle de la fonction cétone est effectuée dans les conditions usuelles. On peut par exemple utiliser l'éthylène glycol en présence d'acide paratoluène sulfonique pour préparer le cétal cyclique.

La réduction éventuelle des produits de formule (X) est effectuée de préférence en présence d'un hydrure tel que l'hydrure de lithium aluminium ou le borohydrure de sodium. L'acylation éventuelle est effectuée dans les conditions indiquées ci-dessus.

Les produits de formule (V) sont décrits dans les brevets américains USP 3.062.845 et 3.775.443. On préfère les produits dans lesquels Alk représente un radical méthyle.

Les exemples suivants illustrent l'invention sans toute-fois la limiter.

**EXEMPLE 1 : Spiro (cyclopentane-1,17'béta-estr-4'-ène) 2,3'-dione**

STADE A : (1,2-éthane diyl) acétal cyclique de 3'-méthoxy-spiro (cyclopentane-1,17'béta-estra-1',3',5',(10')-trièn) 2-one

On ajoute 9 $cm^3$ d'orthoformiate d'éthyle et 90 mg d'acide paratoluène sulfonique à une solution de 4,5 g de 3'-méthoxy-spiro (cyclopentane-1,17'béta-estra-1,3,5(10)-trièn) 2-one dans 18 $cm^3$ de chloroforme et 18 $cm^3$ d'éthylène-glycol et chauffe au reflux une heure. On refroidit, alcalinise par quelques $cm^3$ de triéthylamine puis verse dans une solution saturée aqueuse de bicarbonate de sodium. On extrait à l'acétate

d'éthyle, lave à l'eau salée, sèche sur sulfate de magnésium et évapore le solvant. On obtient 6,36 g de cristaux colorés que l'on recristallise dans le minimum d'éther-isopropylique par chaud et froid. Après séchage, on obtient 4 g de produit attendu F = 117-118 °C.

500 mg de ce produit sont recristallisés dans l'éther isopropylique. On récupère 375 mg de cristaux blancs F = 118-119 °C.
$[alpha]_D$ = +26 ° ± 1,5 ° c = 0,95 % dans $CHCl_3$

| Analyse $C_{25}H_{34}O_3$ = 382,55 | | | | |
|---|---|---|---|---|
| Calculé<br>Trouvé | C% | 78,49<br>78,8 | H% | 8,96<br>9,1 |

STADE B : Spiro (cyclopentane-1,17'béta-estr-4'-ène) 2,3'-dione

On ajoute sous atmosphère inerte 3 g de produit obtenu au stade A en solution dans 4 $cm^3$ d'éthanol et 60 $cm^3$ de tétrahydrofuranne anhydre dans 36 $cm^3$ d'ammoniac condensé à -40 °, -50 °C puis 540 mg de lithium. On ajoute ensuite 250 mg de lithium et 2 $cm^3$ d'éthanol. Après la fin de la réaction on distille l'ammoniac, dilue à l'eau et extrait à l'acétate d'éthyle. Ce résidu gommeux est repris à l'éther, la solution est filtrée et l'on obtient après évaporation 3,06 g de produit de réduction que l'on dissout dans un mélange de 60 $cm^3$ d'éthanol, 10 $cm^3$ de chlorure de méthylène et 10 $cm^3$ d'acide chlorhydrique 2N. On laisse une heure à température ambiante, ajoute 10 $cm^3$ d'acide chlorhydrique 2N et chauffe 40 minutes à 55 °C. On refroidit, dilue à l'eau et extrait au chlorure de méthylène. On lave à l'eau, sèche puis évapore. On obtient 3 g de résidu cristallisé que l'on chromatographie sur silice (éluant cyclohexane-acétate d'éthyle (7-3)) et obtient 2 g de produit. Après recristallisation dans un mélange chlorure de méthylène-éther isopropylique on obtient 1,96 g de produit attendu. F = 172 °C.
Infra-rouge ($CHCl_3$)
1721 $cm^{-1}$ : cyclopentanone
1663-1618 $cm^{-1}$ : diénone
Ultra-violet (éthanol)
Maximum à 239 nm epsilon = 18100
Maximum à 304 nm

**PREPARATION : La 3'-méthoxy-spiro (cyclopentane-1,17'béta-estra-1,3,5(10)trièn) 2-one** utilisée au départ de l'exemple 1 a été obtenue comme suit :

STADE 1 : 17alpha-allyl-3-méthoxy-19-nor-pregna-1,3,5(10)-trièn-20-one

On ajoute 1,1 g de lithium à 500 $cm^3$ d'ammoniac condensé puis goutte à goutte 25,9 g de 17béta-acétoxy-3-méthoxy 19-nor-17alpha-pregna-1,3,5(10)-trièn-20-one, en solution dans 120 $cm^3$ de tétrahydrofuranne anhydre. On distille l'ammoniac sous atmosphère inerte et on ajoute à 20 °C, 25 $cm^3$ de bromure d'allyle. On ajoute 30 $cm^3$ de tétrahydrofuranne anhydre et agite 16 heures à température ambiante. On dilue à l'eau, extrait à l'acétate d'éthyle, lave à l'eau, sèche, distille sous pression réduite et obtient 24 g de produit. On recristallise dans un mélange chlorure de méthylène-éthanol, glace, lave à l'éthanol et sèche à 60 °C sous pression réduite. On obtient 13,78 g de produit attendu F = 110 °C. On évapore les liqueurs mères à sec sous pression réduite et reprend par du cyclohexane au reflux, on obtient une dissolution partielle. La phase soluble est chromatographiée sur silice (éluant chlorure de méthylène-cyclohexane (95-5)). On recueille de nouveau 1,5 g de produit attendu dont on a obtenu un échantillon analytique par recristallisation dans le méthanol aqueux, le pentane-cyclohexane et enfin l'éthanol F = 126 °C.
Infra-rouge ($CHCl_3$)
1696 $cm^{-1}$ : CO en position 20
1357 $cm^{-1}$ : $CH_3$
991, 922 et 1642 $cm^{-1}$ : $-CH=CH_2$
Ultra-violet ($CHCl_3$-EtOH)
273 nm : inflexion
273 nm : maximum epsilon = 2000
286 nm : maximum epsilon = 1900

STADE 2 : 3-méthoxy-20-oxo-19-nor-pregna-1,3,5(10)-trièn-17alpha-acétaldéhyde.

On fait barboter de l'oxygène ozonisé dans une solution de 1 g de produit obtenu au stade 1 dans 30 $cm^3$ de chlorure de méthylène refroidie à -65°,-70°C jusqu'à disparition du composé initial. On dégaze par un courant d'argon et ajoute un mélange de 2,5 g de zinc en poudre, 5 $cm^3$ d'acide acétique et 10 $cm^3$ d'eau. On agite le mélange à température ambiante pendant 17 heures, essore le précipité, lave à l'eau puis avec une solution de bicarbonate de sodium. Après séchage et évaporation, on obtient 1 g de produit attendu. On filtre sur silice avec un mélange benzène-acétate d'éthyle (95-5) et recueille 740 mg de produit attendu. Par recristallisations successives dans un mélange chlorure de méthylène-éther isopropylique puis dans le tétrahydrofuranne aqueux on obtient des cristaux purs. F = 138°C.
Infra-rouge ($CHCl_3$)
Absence de $-HC=CH_2$
1724 $cm^{-1}$ : C=O aldéhyde
1688 $cm^{-1}$ : CO en position 20
1501, 1577 et 1610 $cm^{-1}$ : aromatique.

STADE 3 : 3'-méthoxy-spiro (cyclopent-3-èn-1,17'béta-estra-1',3',5'(10')-trièn) 2-one.

On ajoute 5 $cm^3$ de chlorure de méthylène à un mélange de 734 mg de produit obtenu au stade 2 dans 15 $cm^3$ de potasse méthanolique 0,5 N. Après une heure à température ambiante, on dilue à l'eau et extrait au chlorure de méthylène. On lave, sèche et évapore sous pression réduite pour obtenir 700 mg de produit attendu. Après filtration sur silice, on obtient 600 mg de produit purifié, que l'on recristallise successivement dans des mélanges chlorure de méthylène-éther-isopropylique puis chlorure de méthylène-méthanol ; on obtient un produit fondant à 148°C.
Infra-rouge ($CHCl_3$)
1691 $cm^{-1}$ : cétone conjuguée
1610 $cm^{-1}$ : C=C
1595, 1575, 1499 $cm^{-1}$ : aromatique.

STADE 4 : 3'-méthoxy-spiro (cyclopentane-1,17'béta-estra-1',3',5'(10')-trièn) 2-one.

On hydrogène 5 g de produit obtenu au stade 3, dissous dans 50 $cm^3$ de tétrahydrofuranne en présence de 100 mg de palladium à 10 % sur charbon actif et sous une pression de 1300 mBar d'hydrogène. Après une heure d'agitation, on filtre, évapore la solution et obtient 5,1 g de produit attendu que l'on recristallise dans l'éther isopropylique pour obtenir 4,81 g de produit pur F = 112°C.
$[alpha]_D$ = +52° ± 1,5 (c = 1 % dans $CHCl_3$)
Infra-rouge ($CHCl_3$)
1720 $cm^{-1}$ : C=O
1608, 1575, 1500 $cm^{-1}$ : aromatique.
Spectre de masse $M^+ = 338^+$.

**EXEMPLE 2 : Spiro (cyclopentane-1,17'béta-estra-4',9'-diène) 2,3'-dione**

On ajoute à -40°, -50°C une solution de 28,95 g de produit obtenu comme au stade A de l'exemple 1, dans 600 $cm^3$ de tétrahydrofuranne à 360 $cm^3$ d'ammoniac condensé. On ajoute 6 $cm^3$ d'éthanol puis, par petits morceaux 1,07 g de lithium. Après 15 à 20 minutes, on ajoute 0,93 g de lithium et 6 $cm^3$ d'éthanol puis de nouveau deux fois 400 mg de lithium et 3 $cm^3$ d'éthanol. On distille l'ammoniac et dilue à l'eau à température ambiante. On extrait à l'acétate d'éthyle, lave à l'eau, sèche et évapore. On obtient 29,6 g de produit de réduction que l'on traite par 285 $cm^3$ d'acide acétique à 25 % d'eau sous agitation pendant une heure. On ajoute 20 $cm^3$ d'éther éthylique puis laisse trois heures à température ambiante. On verse dans l'ammoniaque diluée, extrait à l'acétate d'éthyle, lave à l'eau, sèche et évapore. On obtient 29 g de produit que l'on dissout dans 290 $cm^3$ de pyridine, refroidit à 4°C et ajoute 29 g de perbromure de pyridinium par fraction. On agite la suspension à 4°C pendant une heure puis abandonne 17 heures à température ambiante. On verse dans l'eau glacée et extrait au chlorure de méthylène. On lave la phase organique à l'acide chlorhydrique dilué, puis à l'eau, sèche et distille. On obtient 34 g de résidu que l'on chromatographie sur silice sous pression (éluant : cyclohexane-acétate d'éthyle (7-3)). On sépare 17 g de produit qui après recristallisation dans le méthanol donne 16,42 g de produit pur.
F = 192°C.

Infra-rouge ($CHCl_3$)
1722 $cm^{-1}$ : cyclopentanone
866, 1609, 1659 $cm^{-1}$ : diénone
Ultra-violet (Ethanol)
Maximum 303 nm epsilon = 21700
Inflexions 213, 228, 235, 246 nm.

**EXEMPLE 3 : Spiro (cyclopentane-1,17'béta-estra-4',9',11'-triène) 2,3'-dione**

STADE A : 3'-(1,2-éthanediyl) acétal cyclique de spiro (cyclopentane-1,17'béta-estra-5'(10'),9'(11')-diène) 2,3'-dione

On ajoute 4 $cm^3$ d'éthylène glycol et 2 g de chlorhydrate de pyridine à une solution de 9,46 g de produit obtenu à l'exemple 2 dans 190 $cm^3$ de chloroforme sec. On chauffe 5 heures au reflux sous agitation et sous azote. Après refroidissement, on ajoute 2 $cm^3$ de triéthylamine puis dilue à l'aide d'une solution aqueuse de bicarbonate de sodium. On décante la phase organique, lave à l'eau, sèche, filtre et obtient 13,4 g de produit brut. Ce produit est redissous dans dans quelques $cm^3$ de chlorure de méthylène puis on dilue à l'éther. On filtre sur hyflosupercel, dilue de nouveau à l'éther éthylique, concentre et glace la suspension obtenue. On essore et obtient 8,145 g de produit attendu. F = 129°C. Par chromatographie des liqueurs mères après évaporation (éluant : cyclohexane-acétate d'éthyle (8-2)). On obtient de nouveau 1,48 g de produit attendu.
Infra-rouge ($CHCl_3$)
1721 $cm^{-1}$ : cyclopentanone
1641, 1616 $cm^{-1}$ : diène.

STADE B : Spiro (cyclopentane-1,17'béta-estra-4',9',11'-triène) 2,3'-dione

On chauffe une heure à 70°C une solution de 3,17 g de produit obtenu au stade A dans 32 $cm^3$ d'acide acétique à 30 % d'eau. On glace, dilue à l'eau, extrait au chlorure de méthylène, lave la phase organique avec une solution aqueuse de bicarbonate de sodium puis à l'eau. On sèche et évapore sous pression réduite et obtient 2,88 g de produit que l'on traite dans 30 $cm^3$ de dioxanne par 2,3 g de dichloro-dicyano-benzoquinone, par agitation pendant 4 heures à température ambiante. On dilue avec une solution aqueuse de thiosulfate de sodium à 10 %, extrait à l'acétate d'éthyle, lave au bicarbonate de sodium, à l'eau puis sèche et distille. On obtient 2,97 g de résine que l'on purifie par chromatographie sur silice (éluant : cyclohexane - acétate d'éthyle (8-2 puis 7-3) et obtient 2,075 g de produit pur. On obtient un échantillon analytique par recristallisation dans un mélange de chlorure de méthylène-éther isopropylique. F = 156°C.
Infra-rouge ($CHCl_3$)
1574-1649 $cm^{-1}$ : triénone
1725 $cm^{-1}$ : cyclopentanone
Ultra-violet (EtOH)
239 nm : maximum epsilon = 6300
340 nm : maximum epsilon = 31200
270 nm : inflexion.

**EXEMPLE 4 : (2ß) 2-hydroxy-spiro (cyclopentane-1,17'béta-estra-4',9'-dièn) 3'-one**

STADE A : (2S) 3'-méthoxy-spiro (cyclopentane-1,17'béta-estra-2',5'(10')-dièn) 2-ol et isomère (2R) correspondant.

On ajoute une solution de 2,04 g de 3'-méthoxy-spiro (cyclopentane-1,17'béta-estra-1',3',5'(10')-trièn) 2-one dans 40 $cm^3$ de tétrahydrofuranne dans un mélange de 400 mg de lithium dans 100 $cm^3$ d'ammoniac à -40°C. On rince l'ampoule avec 5 $cm^3$ de tétrahydrofuranne et 5 $cm^3$ d'alcool tert-butylique. Après 30 minutes d'agitation à -40°C, on distille l'ammoniac, dilue avec une solution aqueuse de chlorure d'ammonium puis extrait à l'acétate d'éthyle. On lave la phase organique, sèche et évapore sous pression réduite. On obtient 2,11 g de produit brut que l'on chromatographie sur silice (éluant : cyclohexane-acétate d'éthyle (8-2) et 1 % de triéthylamine). On obtient 370 mg de produit 2R et 1,375 g de produit 2S. Après recristallisation du produit 2S dans l'éther isopropylique, on obtient 132 mg d'un échantillon analytique.

F = 124 ° C.

| Infra-rouge ($CHCl_3$) | |
|---|---|
| Produit 2S | Produit 2R |
| 1574-1649 $cm^{-1}$ : OH<br>1695-1665 $cm^{-1}$ : les C=C | 3616 $cm^{-1}$ : OH<br>1695-1664 $cm^{-1}$ : les C=C |

<u>STADE B</u> : (2S) 2-hydroxy-spiro (cyclopentane-1,17'béta-estr-5'(10')-èn) 3'-one

On ajoute 1 $cm^3$ d'éther éthylique à une solution de 200 mg de produit 2S obtenu au stade A dans 4 $cm^3$ d'acide acétique à 20 % et laisse réagir 3 heures à température ambiante. On dilue à l'eau, extrait au chlorure de méthylène, lave au bicarbonate de sodium, sèche et évapore sous pression réduite. On obtient 190 mg de produit attendu.
Infra-rouge ($CHCl_3$)
3614 $cm^{-1}$ : OH
1714 $cm^{-1}$ : 3-céto.

<u>STADE C</u> : (2S) 2-hydroxy-spiro (cyclopentane-1,17'béta-estra-4'9'-dièn) 3'-one

On ajoute 200 mg de perbromure de pyridinium à une solution de 185 mg de produit obtenu au stade B dans 3 $cm^3$ de pyridine et agite à température ambiante pendant 15 heures. On dilue à l'eau, extrait au chlorure de méthylène, lave la phase organique à l'acide chlorhydrique 2N puis à l'eau. On sèche, évapore sous pression réduite et obtient 185 mg de produit attendu. Après chromatographie sur silice (éluant : cyclohexane - acétate d'éthyle (1-1)) et recristallisation dans l'éther isopropylique, on obtient 117 mg de produit pur F = 187 ° C.
Infra-rouge ($CHCl_3$)
3616 $cm^{-1}$ : OH
1605-1652 $cm^{-1}$ : diénone.
Ultra-violet (EtOH)
Maximum 216 nm epsilon = 6400
Maximum 304 nm epsilon = 21600.

**<u>EXEMPLE 5</u> : (2R) 2-hydroxy-spiro (cyclopentane-1,17'béta-estra-4',9'-dièn) 3'-one**

On opère d'abord comme au stade B de l'exemple 4 au départ de 370 mg de produit 2R obtenu au stade A de l'exemple 4 et obtient 0,35 g de produit que l'on dissout dans 15 $cm^3$ de pyridine. On ajoute 380 mg de perbromure de pyridinium, opère comme au stade C de l'exemple 4 et obtient après recristallisation dans l'éther isopropylique 200 mg de produit attendu F = 210 ° C.
Infra-rouge ($CHCl_3$)
3612 $cm^{-1}$ : OH
1649-1605 $cm^{-1}$ : diénone.
Ultra-violet
Maximum 218 nm epsilon = 6200
Maximum 308 nm epsilon = 21100.

**<u>EXEMPLE 6 : COMPOSITION PHARMACEUTIOUE</u>**

On a préparé des comprimés répondant à la formule suivante :

| | |
|---|---|
| - produit de l'exemple 2<br>- excipient q.s. pour un comprimé terminé à (détail de l'excipient : talc, amidon, stéarate de magnésium). | 100 microgrammes<br>100 mg |

## ETUDE PHARMACOLOGIQUE

Activité progestomimétique des produits de l'invention.

a) L'activité progestomimétique des produits des exemples 1 et 2 a été étudiée par la méthode des récepteurs hormonaux décrite par J.P. RAYNAUD et Coll. dans "J. Ster. BIOCHEM" 1975, 6, 615-622 et dans "Physiology and Genetics of Reproduction 1975 part A p. 143-160".

La technique est la suivante :

On administre à des lapins impubères 25 microgrammes d'estradiol par voie per-cutanée. Cinq jours après ce traitement, les animaux sont sacrifiés, les utérus sont alors prélevés et homogénéisés dans un tampon trométhamine 10 mM, saccharose 0,25 M, HCl. pH 7,4. L'homogénat est centrifugé à 105 000 g pendant une heure. Le liquide surnageant ou cytosol est alors ajusté de façon à avoir une dilution de 1/50 (poids/volume).

On incube à 0°C pendant deux heures des tubes de même volume de cytosol avec une concentration fixe de 17,21-diméthyl 19-nor-4,9-pregnadiène 3,20-dione tritié, désigné ci-après par produit R tritié en présence ou non d'une concentration croissante de 17,21-diméthyl 19-nor-4,9-pregnadiène 3,20-dione radio-inerte désignée ci-après produit R froid, de progestérone ou de produit à tester.

On détermine au bout de deux heures et au bout de 24 heures la radioactivité du produit R tritié lié par la technique d'absorption au charbon-destran (1,25 % - 0,625 %).

On trace ensuite :

- la droite parallèle à l'axe des abscisses, d'ordonnée

$$I_{50} = \frac{100\,(1 + \frac{Bmin}{Bo})}{2}$$

  Bo étant la quantité maximum de produit R tritié lié, mesurée dans l'incubat contenant uniquement du produit R tritié, Bmin étant la quantité minimum de produit R tritié lié (non spécifique), mesurée dans l'incubat contenant du produit R tritié plus un grand excès de produit R froid (2500 $10^{-9}$ M)
- les courbes représentant les pourcentages de produit R tritié lié $\frac{B}{Bo}$ en fonction du logarithme des concentrations du produit froid ajouté.

Les intersections de la droite $I_{50}$ et des courbes permettent de déterminer les valeurs CP et CX.

CP : concentration de la progestérone froide qui inhibe de 50 % la fixation du produit R tritié.

CX : concentration du produit testé qui inhibe de 50 % la fixation du produit R tritié.

L'affinité relative du produit testé ou ARL est donnée par la formule :

$$ARL = 100 \times \frac{CP}{CX}$$

Résultats :

| | ARL 2 heures | ARL 24 heures |
|---|---|---|
| Progestérone | 100 | 100 |
| Produit de l'exemple 1 | 312 | 1012 |
| Produit de l'exemple 2 | 344 | 912 |

Conclusion : les produits des exemples 1 et 2 présentent une très forte affinité pour le récepteur spécifique de la progestérone.

b) L'activité progestomimétique du produit de l'exemple 2, a été déterminée sur le test de CLAUBERG. Selon ce test, des groupes de trois lapines impubères sont préalablement sensibilisées par administration d'estradiol, par voie sous-cutanée, pendant cinq jours, à la dose quotidienne de 5 microgrammes, deux jours plus tard elles sont traitées quotidiennement, pendant cinq jours, avec le médicament étudié, par voie sous-cutanée. Les animaux sont sacrifiés le sixième jour et sur les coupes de l'utérus, la

prolifération en dentelle de l'endomètre, caractéristique de l'action progestomimétique, est notée en unités Mac Phail.

Le produit de l'exemple 2 est utilisé en solution dans l'huile de sésame additionnée de 5 % d'alcool benzylique.

| TRAITEMENT par | DOSES microgrammes/lapin/jour | UNITES Mac Phail |
|---|---|---|
| Produit de l'exemple 2 | 0,3<br>1<br>3 | 0,5<br>1,8<br>2,8 |

On a donc obtenu 2,8 unités Mac Phail à la dose quotidienne de 10 microgrammes. Le produit étudié possède donc une très forte activité progestomimétique.

**Revendications**

**Revendications pour les Etats contractants suivants : AT, BE, CH, DE, DK, FR, GB, IT, LI, LU, NL, SE**

**1.** Les produits de formule générale (I) :

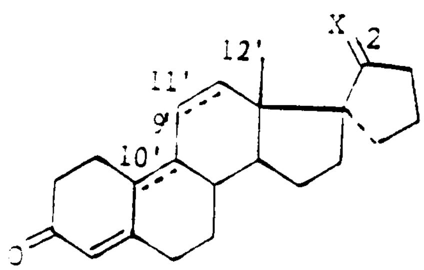

(I)

dans laquelle =X représente un fonction cétonique ou un

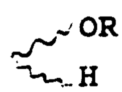

radical dans lequel R représente un atome d'hydrogène ou un radical acyle ayant au plus 12 atomes de carbone et les traits ondulés signifient que les substituants OR et hydrogène peuvent se trouver en positions alpha ou béta,
les traits pointillés en positions 9'(10') et 11'(12'), indiquent la présence éventuelle d'une seconde double liaison en position 9'(10') ou de deux double liaisons supplémentaires en positions 9'(10') et 11'-(12'), sous forme de chacun des épimères 2R ou 2S ou sous forme d'un mélange de ces épimères, le trait pointillé en position 17 indique que la liaison est en position alpha.

**2.** Les produits de formule générale (I) telle que définie à la revendication 1 dans laquelle X représente un atome d'oxygène et le trait pointillé en position 11'(12') n'indique pas la présence d'une seconde liaison entre les carbones qui le portent.

**3.** Le spiro (cyclopentane-1,17'béta-estra-4',9'-diène) 2,3'-dione.

**4.** Procédé de préparation des produits de formule générale (I) telle que définie à la revendication 1 caractérisé en ce que l'on soumet un produit de formule (II) :

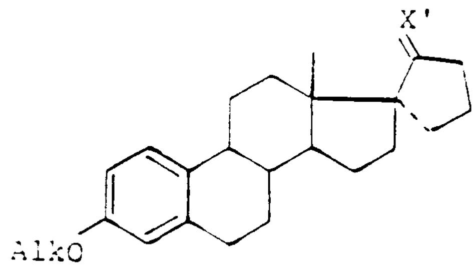

(II)

dans laquelle =X' représente une fonction cétonique éventuellement protégée ou un radical

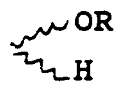

R ayant la définition indiquée à la revendication 1, sous forme de chacun des épimères 2R ou 2S ou sous forme d'un mélange de ces épimères et Alk représente un radical alkyle ayant de 1 à 4 atomes de carbone
soit à une réduction suivie d'une hydrolyse avec un acide fort pour obtenir les produits de formule ($I_a$) :

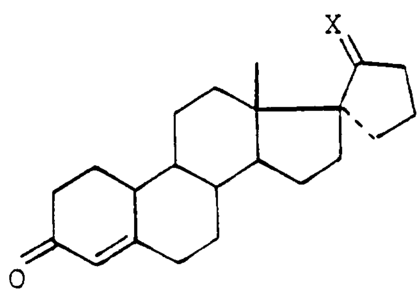

($I_a$)

dans laquelle X a la signification indiquée à la revendication 1 et correspondant aux produits de formule (I) dans laquelle les traits pointillés en position 9'(10') et 11'(12') ne représentent pas une seconde liaison entre les carbones qui les portent,
soit à une réduction suivie d'une hydrolyse douce avec un acide faible puis d'une déshydrogénation pour obtenir les produits de formule ($I_b$) :

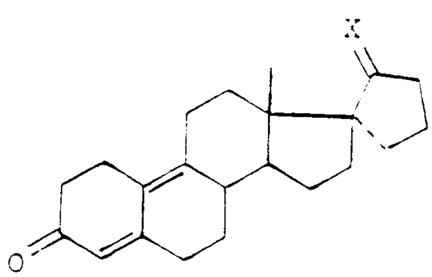

($I_b$)

correspondant aux produits de formule (I) dans laquelle les traits pointillés en positions 9'(10') indiquent la présence d'une double liaison et ceux en position 11'(12') ne représentent pas de double liaison, et produits de formule ($I_b$) que l'on soumet éventuellement à une réaction de protection en position 3, suivie d'une hydrolyse douce avec un acide faible puis d'une déshydrogénation pour obtenir les produits de formule ($I_c$) :

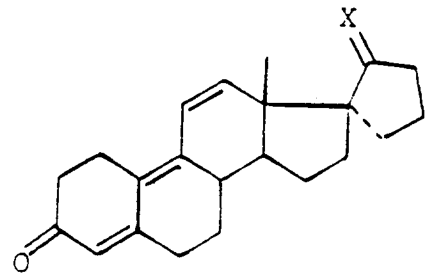

$(I_c)$

correspondant aux produits de formule (I) dans laquelle les traits pointillés en positions 9'(10') et 11'-(12') indiquent la présence de deux double liaisons supplémentaires en position 9'(10') et 11'(12'),
et si désiré, soumet les produits de formules $(I_a)$, $(I_b)$ et $(I_c)$ indiquées ci-dessus et dans lesquelles =X représente un radical

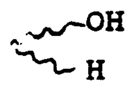

soit à une réaction d'oxydation pour obtenir les produits de formules $(I_a)$, $(I_b)$ et $(I_c)$ correspondants dans lesquels X représente un atome d'oxygène,
soit à une réaction d'acylation pour obtenir les produits dans lesquels =X représente un radical

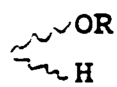

dans lequel R représente un radical acyle.

**5.** Procédé selon la revendication 4 pour la préparation des produits de formule (I') correspondant aux produits de formule générale (I) telle que décrite à la revendication 1 dans laquelle =X représente un radical

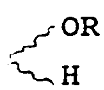

R ayant la définition indiquée à la revendication 1, caractérisé en ce que l'on soumet un produit de formule (II') :

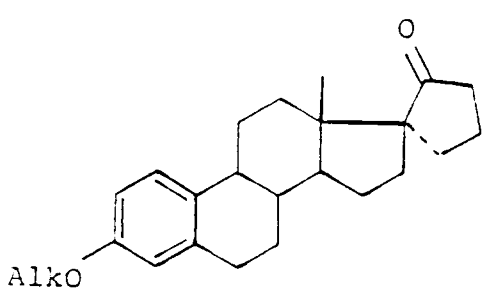

(II')

à une réaction de réduction pour obtenir les produits de formule (III) :

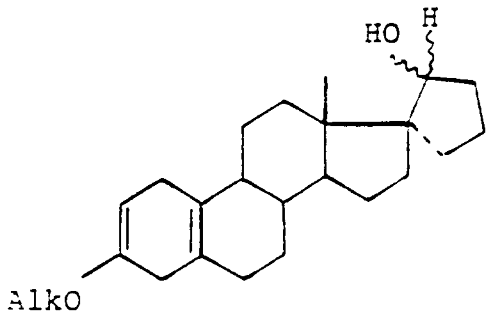

(III)

produits de formule (III) que l'on sépare si désiré en chacun de leurs isomères et soumet les produits de formule (III) sous forme de mélange d'épimères ou sous forme de produits purs

soit à une hydrolyse avec un acide fort pour obtenir les produits de formule ($I'_a$) :

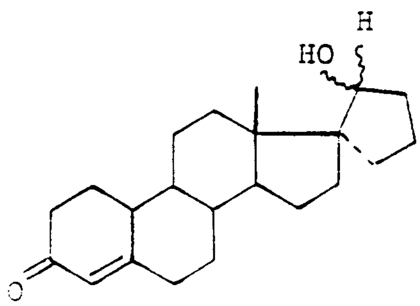

($I'_a$)

produits de formule ($I'_a$) que l'on sépare si désiré en chacun de leurs isomères,

soit à une hydrolyse douce avec un acide faible suivie d'une déshydrogénation pour obtenir les produits de formule ($I'_b$) :

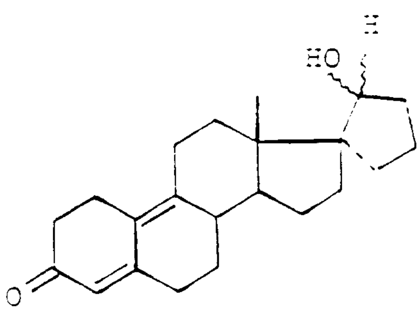

($I'_b$)

produits de formule ($I'_b$) que l'on sépare si désiré en chacun de leurs isomères et produits de formule ($I'_b$) sous forme de mélange d'épimères ou sous forme de produits purs que l'on soumet éventuellement à une réaction de protection en position 3 suivie d'une hydrolyse douce avec un acide faible puis d'une déshydrogénation pour obtenir les produits de formule ($I'_c$) :

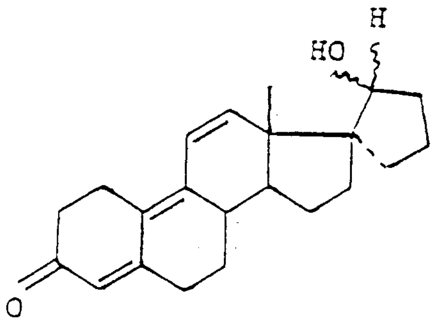

(I'c)

produits de formule (I'$_c$) que l'on sépare si désiré en chacun de leurs isomères et soumet si désiré les produits de formule (I'$_a$), (I'$_b$) et (I'$_c$) ou le cas échéant leurs isomères à une réaction d'acylation pour obtenir les produits correspondants dans lesquels =X représente un radical

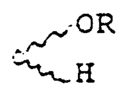

dans lequel R représente un radical acyle.

6. A titre de médicaments les produits de formule (I) telle que définie à l'une des revendications 1 ou 2.

7. A titre de médicament le produit défini à la revendication 3.

8. Les compositions pharmaceutiques renfermant, comme principe actif, au moins un médicament tel que défini à l'une des revendications 6 ou 7.

9. A titre de produits industriels nouveaux, les produits de formules (II) et (III),

II

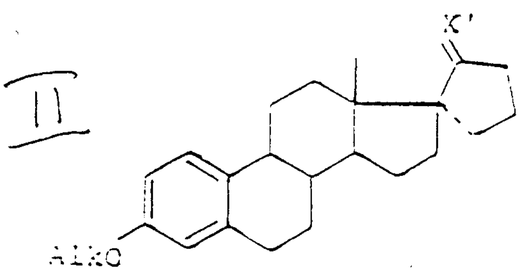

III

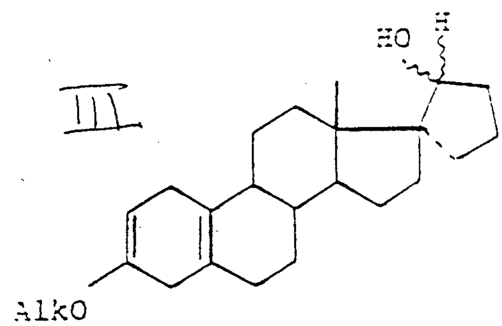

dans lesquelles = X' représente une fonction cétonique éventuellement protégée ou un radical

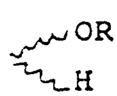

R ayant la définition indiquée à la revendication 1, sous forme de chacun des épimères 2R ou 2S ou sous forme d'un mélange de ces épimères et Alk représente un radical alkyle ayant de 1 à 4 atomes de carbone.

**Revendications pour l'Etat contractant suivant : ES**

**1.** Procédé de préparation des produits de formule générale (I) :

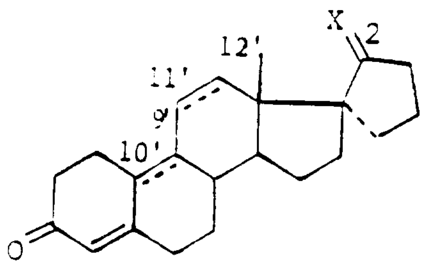

(I)

dans laquelle =X représente un fonction cétonique ou un

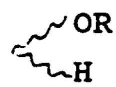

radical dans lequel R représente un atome d'hydrogène ou un radical acyle ayant au plus 12 atomes de carbone et les traits ondulés signifient que les substituants OR et hydrogène peuvent se trouver en positions alpha ou béta,
les traits pointillés en positions 9'(10') et 11'(12'), indiquent la présence éventuelle d'une seconde double liaison en position 9'(10') ou de deux double liaisons supplémentaires en positions 9'(10') et 11'-(12'), sous forme de chacun des épimères 2R ou 2S ou sous forme d'un mélange de ces épimères, le trait pointillé en position 17 indique que la liaison est en position alpha, caractérisé en ce que l'on soumet un produit de formule (II) :

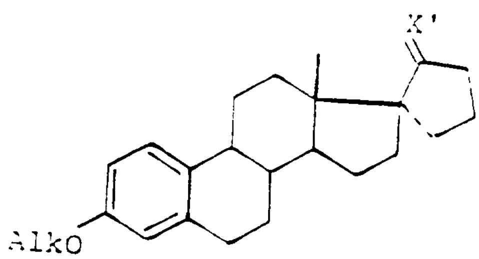

(II)

dans laquelle =X' représente une fonction cétonique éventuellement protégée ou un radical

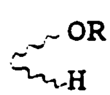

R ayant la définition indiquée ci-dessus, sous forme de chacun des épimères 2R ou 2S ou sous forme d'un mélange de ces épimères et Alk représente un radical alkyle ayant de 1 à 4 atomes de carbone
soit à une réduction suivie d'une hydrolyse avec un acide fort pour obtenir les produits de formule ($I_a$) :

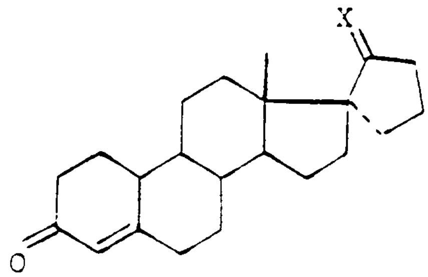

$(I_a)$

dans laquelle X a la signification indiquée ci-dessus, et correspondant aux produits de formule (I) dans laquelle les traits pointillés en position 9'(10') et 11'(12') ne représentent pas une seconde liaison entre les carbones qui les portent,
<u>soit</u> à une réduction suivie d'une hydrolyse douce avec un acide faible puis d'une déshydrogénation pour obtenir les produits de formule $(I_b)$ :

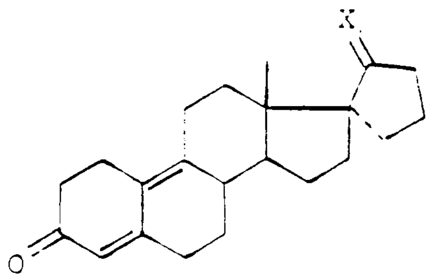

$(I_b)$

correspondant aux produits de formule (I) dans laquelle les traits pointillés en positions 9'(10') indiquent la présence d'une double liaison et ceux en position 11'(12') ne représentent pas de double liaison, et produits de formule $(I_b)$ que l'on soumet éventuellement à une réaction de protection en position 3, suivie d'une hydrolyse douce avec un acide faible puis d'une déshydrogénation pour obtenir les produits de formule $(I_c)$ :

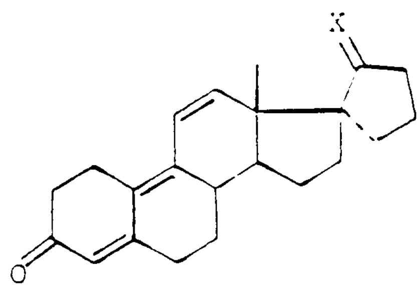

$(I_c)$

correspondant aux produits de formule (I) dans laquelle les traits pointillés en positions 9'(10') et 11'-(12') indiquent la présence de deux double liaisons supplémentaires en position 9'(10') et 11'(12'),
et si désiré, soumet les produits de formules $(I_a)$, $(I_b)$ et $(I_c)$ indiquées ci-dessus et dans lesquelles =X représente un radical

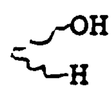

<u>soit</u> à une réaction d'oxydation pour obtenir les produits de formules $(I_a)$, $(I_b)$ et $(I_c)$ correspondants dans lesquels X représente un atome d'oxygène,
<u>soit</u> à une réaction d'acylation pour obtenir les produits dans lesquels =X représente un radical

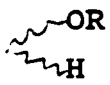

dans lequel R représente un radical acyle

2. Procédé selon la revendication 1 pour la préparation des produits de formule (I') correspondant aux produits de formule générale (I) telle que décrite à la revendication 1 dans laquelle =X représente un radical

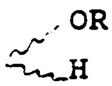

R ayant la définition indiquée à la revendication 1, caractérisé en ce que l'on soumet un produit de formule (II') :

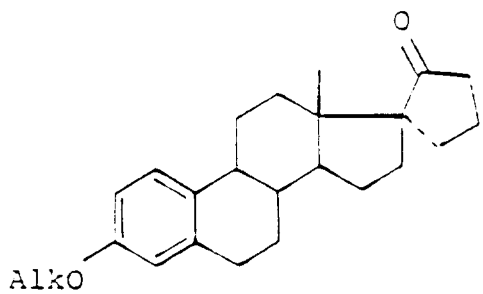

(II')

à une réaction de réduction pour obtenir les produits de formule (III) :

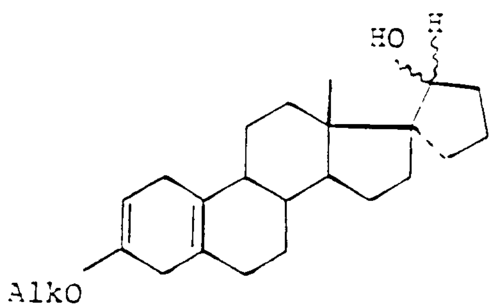

(III)

produits de formule (III) que l'on sépare si désiré en chacun de leurs isomères et soumet les produits de formule (III) sous forme de mélange d'épimères ou sous forme de produits purs
soit à une hydrolyse avec un acide fort pour obtenir les produits de formule ($I'_a$) :

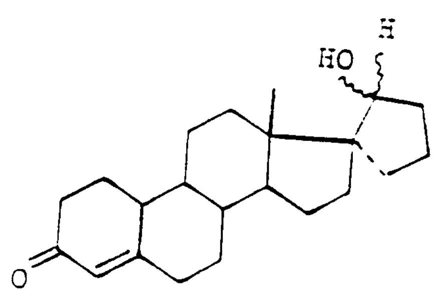

($I'_a$)

produits de formule ($I'_a$) que l'on sépare si désiré en chacun de leurs isomères,
soit à une hydrolyse douce avec un acide faible suivie d'une déshydrogénation pour obtenir les

produits de formule ($I'_b$) :

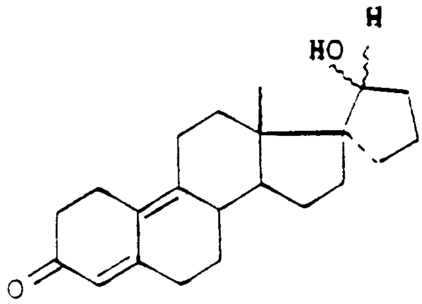

($I'_b$)

produits de formule ($I'_b$) que l'on sépare si désiré en chacun de leurs isomères et produits de formule ($I'_b$) sous forme de mélange d'épimères ou sous forme de produits purs que l'on soumet éventuellement à une réaction de protection en position 3 suivie d'une hydrolyse douce avec un acide faible puis d'une déshydrogénation pour obtenir les produits de formule ($I'_c$) :

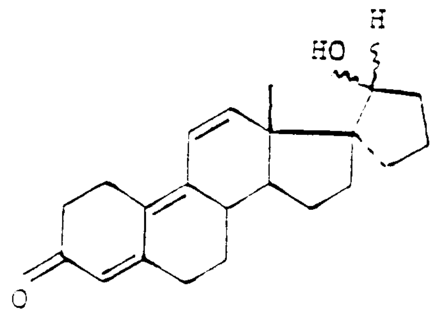

($I'_c$)

produits de formule ($I'_c$) que l'on sépare si désiré en chacun de leurs isomères et soumet si désiré les produits de formule ($I'_a$), ($I'_b$) et ($I'_c$) ou le cas échéant leurs isomères à une réaction d'acylation pour obtenir les produits correspondants dans lesquels =X représente un radical

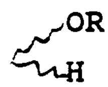

dans lequel R représente un radical acyle.

**Revendications pour l'Etat contractant suivant : GR**

**1.** Procédé de préparation des produits de formule générale (I) :

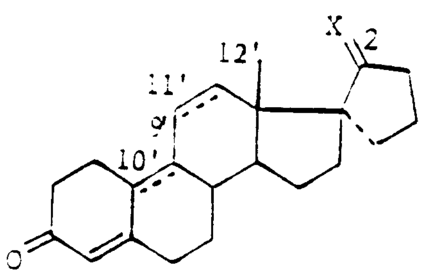

(I)

dans laquelle =X représente un fonction cétonique ou un

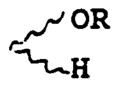

radical dans lequel R représente un atome d'hydrogène ou un radical acyle ayant au plus 12 atomes de carbone et les traits ondulés signifient que les substituants OR et hydrogène peuvent se trouver en positions alpha ou béta,
les traits pointillés en positions 9'(10') et 11'(12'), indiquent la présence éventuelle d'une seconde double liaison en position 9'(10') ou de deux double liaisons supplémentaires en positions 9'(10') et 11'-(12'), sous forme de chacun des épimères 2R ou 2S ou sous forme d'un mélange de ces épimères, le trait pointillé en position 17 indique que la liaison est en position alpha, caractérisé en ce que l'on soumet un produit de formule (II) :

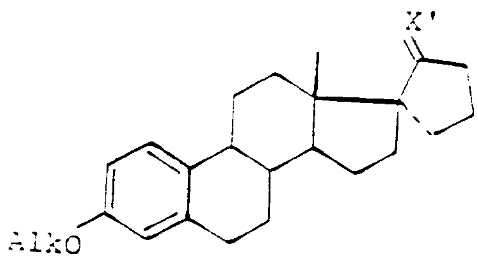

(II)

dans laquelle =X' représente une fonction cétonique éventuellement protégée ou un radical

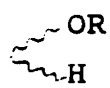

R ayant la définition indiquée ci-dessus, sous forme de chacun des épimères 2R ou 2S ou sous forme d'un mélange de ces épimères et Alk représente un radical alkyle ayant de 1 à 4 atomes de carbone
soit à une réduction suivie d'une hydrolyse avec un acide fort pour obtenir les produits de formule ($I_a$) :

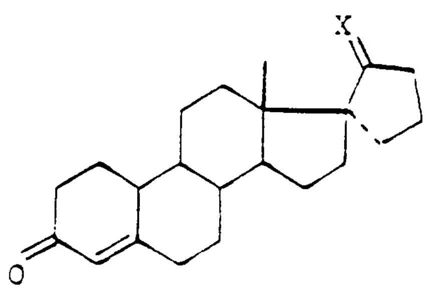

($I_a$)

dans laquelle X a la signification indiquée ci-dessus, et correspondant aux produits de formule (I) dans laquelle les traits pointillés en position 9'(10') et 11'(12') ne représentent pas une seconde liaison entre les carbones qui les portent,
soit à une réduction suivie d'une hydrolyse douce avec un acide faible puis d'une déshydrogénation pour obtenir les produits de formule ($I_b$) :

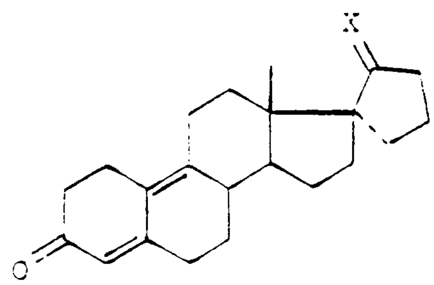

($I_b$)

correspondant aux produits de formule (I) dans laquelle les traits pointillés en positions 9'(10') indiquent la présence d'une double liaison et ceux en position 11'(12') ne représentent pas de double liaison, et produits de formule ($I_b$) que l'on soumet éventuellement à une réaction de protection en position 3, suivie d'une hydrolyse douce avec un acide faible puis d'une déshydrogénation pour obtenir les produits de formule ($I_c$) :

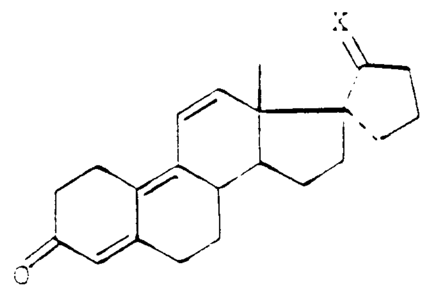

($I_c$)

correspondant aux produits de formule (I) dans laquelle les traits pointillés en positions 9'(10') et 11'-(12') indiquent la présence de deux double liaisons supplémentaires en position 9'(10') et 11'(12'), et si désiré, soumet les produits de formules ($I_a$), ($I_b$) et ($I_c$) indiquées ci-dessus et dans lesquelles =X représente un radical

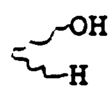

soit à une réaction d'oxydation pour obtenir les produits de formules ($I_a$), ($I_b$) et ($I_c$) correspondants dans lesquels X représente un atome d'oxygène,
soit à une réaction d'acylation pour obtenir les produits dans lesquels =X représente un radical

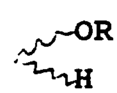

dans lequel R représente un radical acyle.

**2.** Procédé selon la revendication 1 pour la préparation des produits de formule (I') correspondant aux produits de formule générale (I) telle que décrite à la revendication 1 dans laquelle =X représente un radical

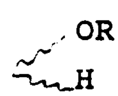

R ayant la définition indiquée à la revendication 1, caractérisé en ce que l'on soumet un produit de formule (II') :

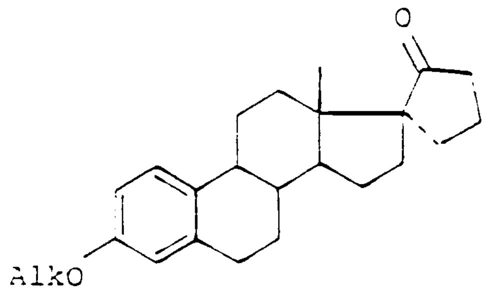

(II')

à une réaction de réduction pour obtenir les produits de formule (III) :

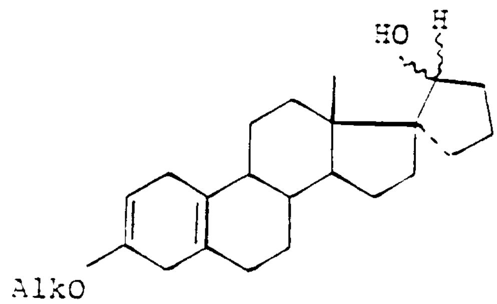

(III)

produits de formule (III) que l'on sépare si désiré en chacun de leurs isomères et soumet les produits de formule (III) sous forme de mélange d'épimères ou sous forme de produits purs
soit à une hydrolyse avec un acide fort pour obtenir les produits de formule (I'$_a$) :

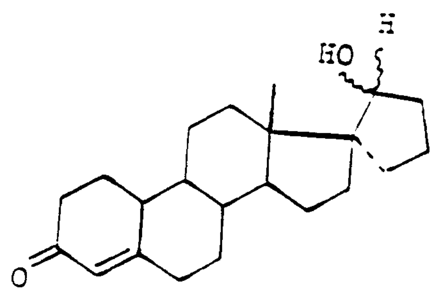

(I'$_a$)

produits de formule (I'$_a$) que l'on sépare si désiré en chacun de leurs isomères,
soit à une hydrolyse douce avec un acide faible suivie d'une déshydrogénation pour obtenir les produits de formule (I'$_b$) :

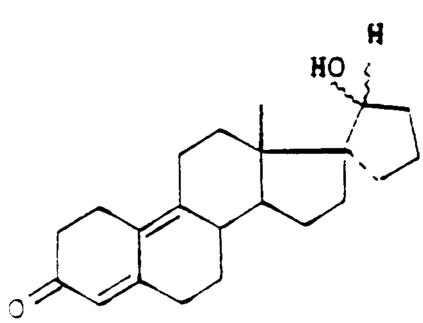

(I'$_b$)

produits de formule ($I'_b$) que l'on sépare si désiré en chacun de leurs isomères et produits de formule ($I'_b$) sous forme de mélange d'épimères ou sous forme de produits purs que l'on soumet éventuellement à une réaction de protection en position 3 suivie d'une hydrolyse douce avec un acide faible puis d'une déshydrogénation pour obtenir les produits de formule ($I'_c$) :

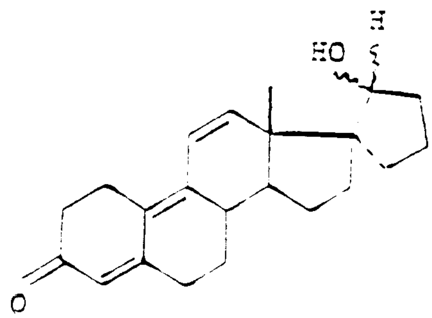

($I'_c$)

produits de formule ($I'_c$) que l'on sépare si désiré en chacun de leurs isomères et soumet si désiré les produits de formule ($I'_a$), ($I'_b$) et ($I'_c$) ou le cas échéant leurs isomères à une réaction d'acylation pour obtenir les produits correspondants dans lesquels =X représente un radical

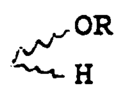

dans lequel R représente un radical acyle.

3. Procédé selon la revendication 1, caractérisé en ce que l'on prépare le spiro (cyclopentane-1,17'béta-estra-4',9'-diène) 2,3'-dione.

4. Procédé de préparation de compositions pharmaceutiques, caractérisé en ce que l'on met à titre de principe actif l'un au moins des produits de formule (I) pharmaceutiquement acceptables, tels que définis à la revendication 1 ou 2 sous une forme destinée à cet usage.

5. Procédé de préparation de compositions pharmaceutiques, caractérisé en ce que l'on met à titre de principe actif l'un au moins des produits de formule (I) pharmaceutiquement acceptables, tels que définis à la revendication 3 sous une forme destinée à cet usage.

**Claims**
**Claims for the following Contracting States : AT, BE, CH, DE, DK, FR, GB, IT, LI, LU, NL, SE**

1. The products of general formula (I):

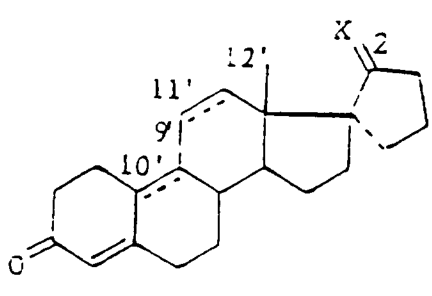

(I)

in which =X represents a ketone function or a

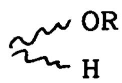

radical in which R represents a hydrogen atom or an acyl radical having at most 12 carbon atoms and the wavy lines indicate that the OR and hydrogen substituents can be found in the alpha or beta positions,
the dotted lines in positions 9'(10') and 11'(12') indicate the optional presence of a second double bond in position 9'(10') or of two additional double bonds in positions 9'(10') and 11'(12'), in the form of each of the epimers 2R or 2S or in the form of a mixture of these epimers, the dotted line in position 17 indicates that the bond is in the alpha position.

2. The products of general formula (I) as defined in claim 1 in which X represents an oxygen atom and the dotted line in position 11'(12') does not indicate the presence of a second bond between the carbons that carry it.

3. Spiro (cyclopentane-1,17'beta-estra-4',9'-diene) 2,3'-dione.

4. Preparation process for the products of general formula (I) as defined in claim 1, characterized in that a product of formula (II):

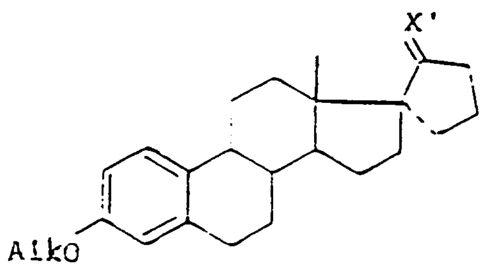

(II)

in which =X' represents an optionally protected ketone function or a

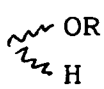

radical, R having the definition indicated in claim 1, in the form of each of the epimers 2R or 2S or in the form of a mixture of these epimers and Alk represents an alkyl radical having 1 to 4 carbon atoms, is subjected
either to a reduction followed by a hydrolysis with a strong acid in order to obtain the products of formula ($I_a$):

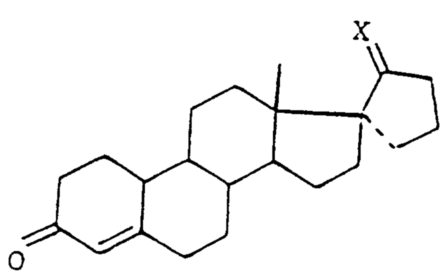

($I_a$)

in which X has the meaning indicated in claim 1 and corresponding to the products of formula (I) in which the dotted lines in position 9'(10') and 11'(12') do not represent a second bond between the carbons that carry them,

or to a reduction followed by a gentle hydrolysis with a weak acid then a dehydrogenation in order to obtain the products of formula ($I_b$):

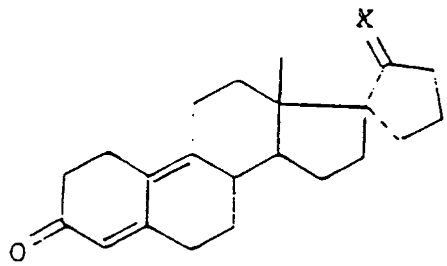

($I_b$)

corresponding to the products of formula (I) in which the dotted lines in position 9'(10') indicate the presence of a double bond and those in position 11'(12') do not represent a double bond, and which products of formula ($I_b$) are optionally subjected to a protection reaction in position 3, followed by a gentle hydrolysis with a weak acid then a dehydrogenation in order to obtain the products of formula ($I_c$):

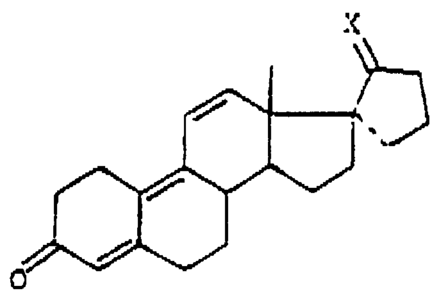

($I_c$)

corresponding to the products of formula (I) in which the dotted lines in positions 9'(10') and 11'(12') indicate the presence of two additional double bonds in position 9'(10') and 11'(12'),
and if desired, the products of formulae ($I_a$), ($I_b$) and ($I_c$) indicated above and in which =X represents a

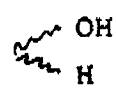

radical are subjected
either to an oxidation reaction in order to obtain the corresponding products of formulae ($I_a$), ($I_b$) and ($I_c$) in which X represents an oxygen atom,
or to an acylation reaction in order to obtain the products in which =X represents a

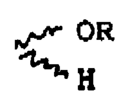

radical in which R represents an acyl radical.

**5.** Process according to claim 4 for the preparation of the products of formula (I') corresponding to the products of general formula (I) as described in claim 1 in which =X represents a

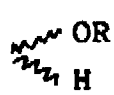

radical, R having the definition indicated in claim 1, characterized in that a product of formula (II'):

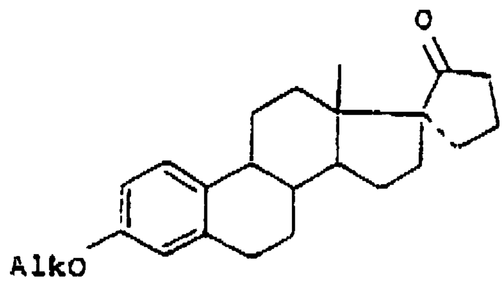

(II')

is subjected to a reduction reaction in order to obtain the products of formula (III):

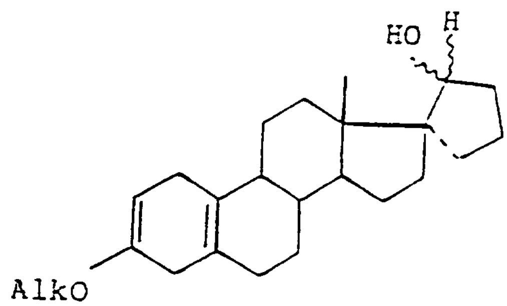

(III)

which products of formula (III) are separated if desired into each of their isomers and the products of formula (III) in the form of epimer mixtures or in the form of pure products are subjected
either to a hydrolysis with a strong acid in order to obtain the products of formula ($I'_a$):

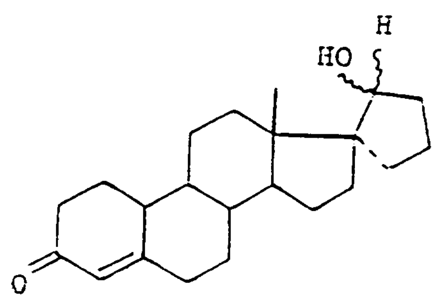

($I'_a$)

which products of formula ($I'_a$) are separated if desired into each of their isomers,
or to a gentle hydrolysis with a weak acid followed by a dehydrogenation in order to obtain the products of formula ($I'_b$):

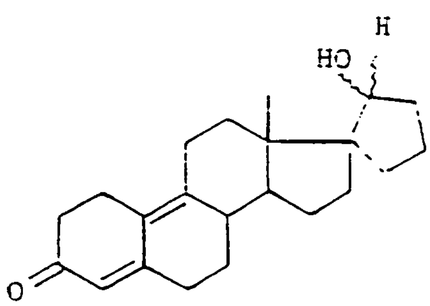

($I'_b$)

which products of formula ($I'_b$) are separated if desired into each of their isomers and which products of formula ($I'_b$) in the form of epimer mixtures or in the form of pure products are optionally subjected to a protection reaction in position 3 followed by a gentle hydrolysis with a weak acid then a dehydrogenation in order to obtain the products of formula ($I'_c$):

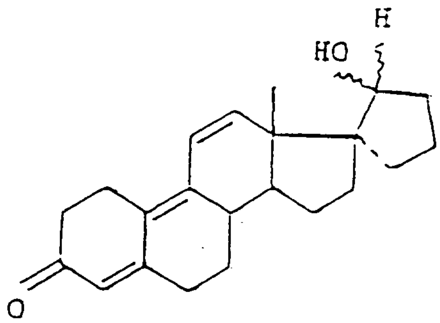

($I'_c$)

which products of formula ($I'_c$) are separated if desired into each of their isomers and if desired the products of formulae ($I'_a$), ($I'_b$) and ($I'_c$) or if appropriate their isomers are subjected to an acylation reaction in order to obtain the corresponding products in which =X represents a

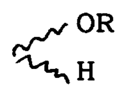

radical in which R represents an acyl radical.

**6.** As medicaments, the products of formula (I) as defined in one of claims 1 or 2.

**7.** As a medicament, the product defined in claim 3.

**8.** Pharmaceutical compositions containing, as active ingredient, at least one medicament as defined in one of claims 6 or 7.

**9.** As new industrial products, the products of formulae (II) and (III):

(II)

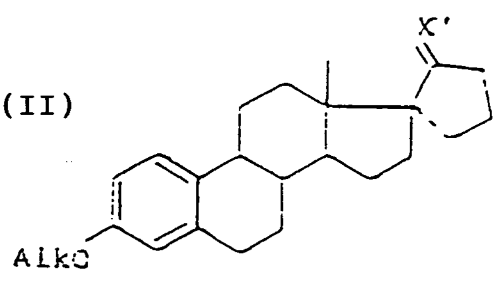

(III)

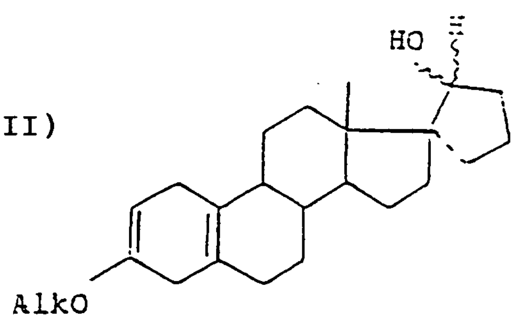

in which =X' represents an optionally protected ketone function or a

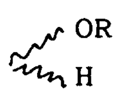

radical, R having the definition indicated in claim 1, in the form of each of the epimers 2R or 2S or in the form of a mixture of these epimers and Alk represents an alkyl radical having 1 to 4 carbon atoms.

**Claims for the following Contracting State : ES**

**1.** Preparation process for the products of general formula (I):

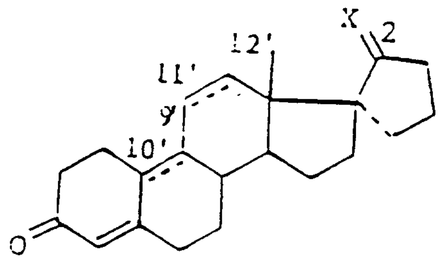

(I)

in which =X represents a ketone function or a

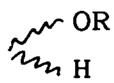

radical in which R represents a hydrogen atom or an acyl radical having at most 12 carbon atoms and the wavy lines indicate that the OR and hydrogen substituents can be found in the alpha or beta positions,
the dotted lines in positions 9'(10') and 11'(12') indicate the optional presence of a second double bond in position 9'(10') or of two additional double bonds in positions 9'(10') and 11'(12'), in the form of each of the epimers 2R or 2S or in the form of a mixture of these epimers, the dotted line in position 17 indicates that the bond is in the alpha position, characterized in that a product of formula (II):

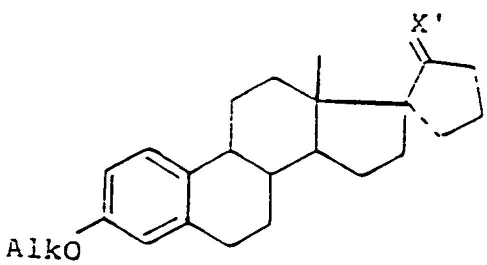

(II)

in which =X' represents an optionally protected ketone function or a

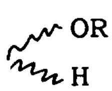

radical, R having the definition indicated in claim 1, in the form of each of the epimers 2R or 2S or in the form of a mixture of these epimers and Alk represents an alkyl radical having 1 to 4 carbon atoms, is subjected
<u>either</u> to a reduction followed by a hydrolysis with a strong acid in order to obtain the products of formula ($I_a$):

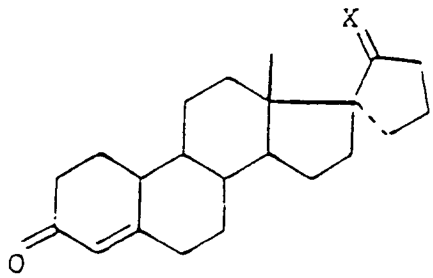

(Ia)

in which X has the meaning indicated in claim 1 and corresponding to the products of formula (I) in which the dotted lines in position 9'(10') and 11'(12') do not represent a second bond between the carbons that carry them,
or to a reduction followed by a gentle hydrolysis with a weak acid then a dehydrogenation in order to obtain the products of formula ($I_b$):

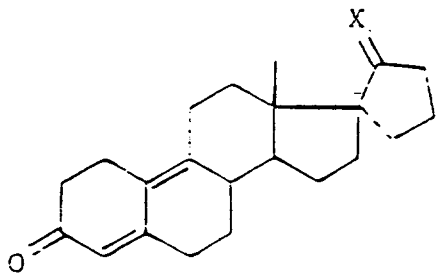

($I_b$)

corresponding to the products of formula (I) in which the dotted lines in position 9'(10') indicate the presence of a double bond and those in position 11'(12') do not represent a double bond, and which products of formula ($I_b$) are optionally subjected to a protection reaction in position 3, followed by a gentle hydrolysis with a weak acid then a dehydrogenation in order to obtain the products of formula ($I_c$):

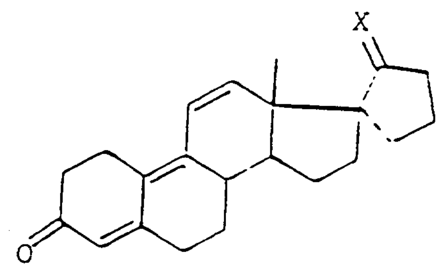

($I_c$)

corresponding to the products of formula (I) in which the dotted lines in positions 9'(10') and 11'(12') indicate the presence of two additional double bonds in position 9'(10') and 11'(12'),
and if desired, the products of formulae ($I_a$), ($I_b$) and ($I_c$) indicated above and in which =X represents a

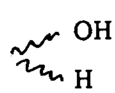

radical are subjected
either to an oxidation reaction in order to obtain the corresponding products of formulae ($I_a$), ($I_b$) and ($I_c$) in which X represents an oxygen atom,
or to an acylation reaction in order to obtain the products in which =X represents a

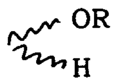

radical in which R represents an acyl radical.

2. Process according to claim 1 for the preparation of the products of formula (I') corresponding to the products of general formula (I) as described in claim 1 in which =X represents a

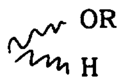

radical, R having the definition indicated in claim 1, characterized in that a product of formula (II'):

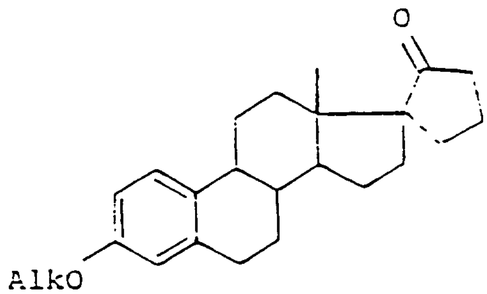

(II')

is subjected to a reduction reaction in order to obtain the products of formula (III):

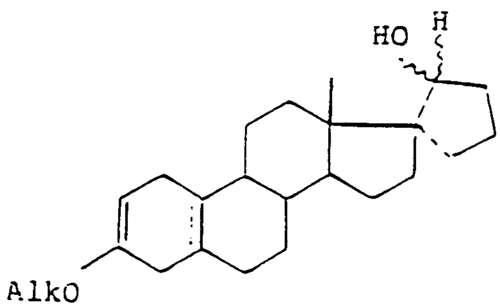

(III)

which products of formula (III) are separated if desired into each of their isomers and the products of formula (III) in the form of epimer mixtures or in the form of pure products are subjected
<u>either</u> to a hydrolysis with a strong acid in order to obtain the products of formula ($I'_a$):

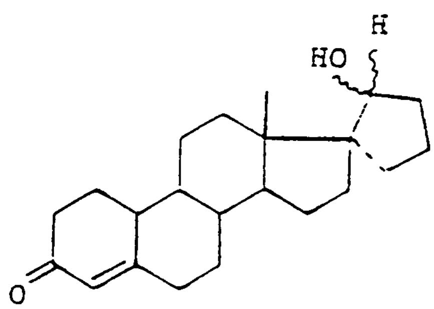

($I'_a$)

which products of formula ($I'_a$) are separated if desired into each of their isomers,
<u>or</u> to a gentle hydrolysis with a weak acid followed by a dehydrogenation in order to obtain the products of formula ($I'_b$):

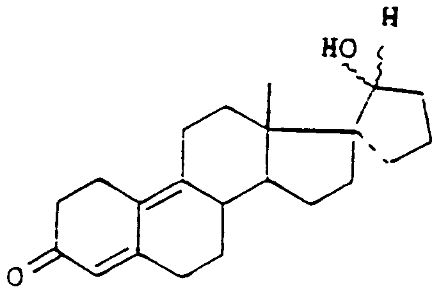

(I'$_b$)

which products of formula (I'$_b$) are separated if desired into each of their isomers and which products of formula (I'$_b$) in the form of epimer mixtures or in the form of pure products are optionally subjected to a protection reaction in position 3 followed by a gentle hydrolysis with a weak acid then a dehydrogenation in order to obtain the products of formula (I'$_c$):

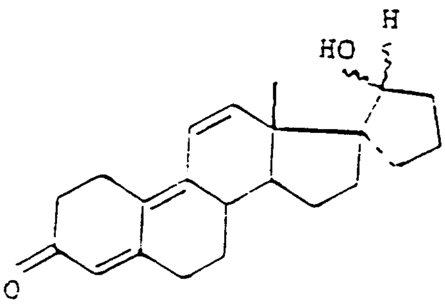

(I'$_c$)

which products of formula (I'$_c$) are separated if desired into each of their isomers and if desired the products of formulae (I'$_a$), (I'$_b$) and (I'$_c$) or if appropriate their isomers are subjected to an acylation reaction in order to obtain the corresponding products in which =X represents a

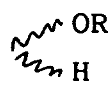

radical in which R represents an acyl radical.

**Claims for the following Contracting State : GR**

**1.** Preparation process for the products of general formula (I):

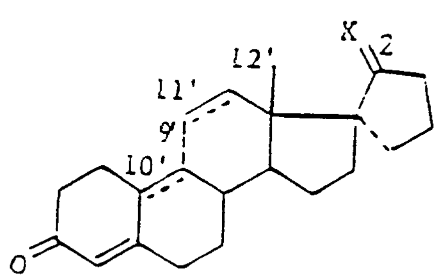

(I)

in which =X represents a ketone function or a

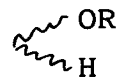

radical in which R represents a hydrogen atom or an acyl radical having at most 12 carbon atoms and the wavy lines indicate that the OR and hydrogen substituents can be found in the alpha or beta positions,
the dotted lines in positions 9'(10') and 11'(12') indicate the optional presence of a second double bond in position 9'(10') or of two additional double bonds in positions 9'(10') and 11'(12'), in the form of each of the epimers 2R or 2S or in the form of a mixture of these epimers, the dotted line in position 17 indicates that the bond is in the alpha position, characterized in that a product of formula (II):

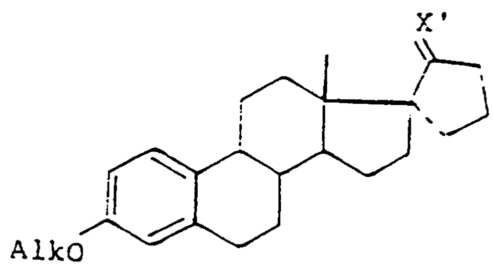

(II)

in which =X' represents an optionally protected ketone function or a

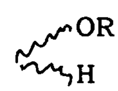

radical, R having the definition indicated in claim 1, in the form of each of the epimers 2R or 2S or in the form of a mixture of these epimers and Alk represents an alkyl radical having 1 to 4 carbon atoms, is subjected
either to a reduction followed by a hydrolysis with a strong acid in order to obtain the products of formula ($I_a$):

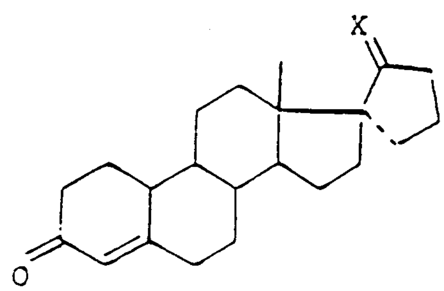

($I_a$)

in which X has the meaning indicated in claim 1 and corresponding to the products of formula (I) in which the dotted lines in position 9'(10') and 11'(12') do not represent a second bond between the carbons that carry them,
or to a reduction followed by a gentle hydrolysis with a weak acid then a dehydrogenation in order to obtain the products of formula ($I_b$):

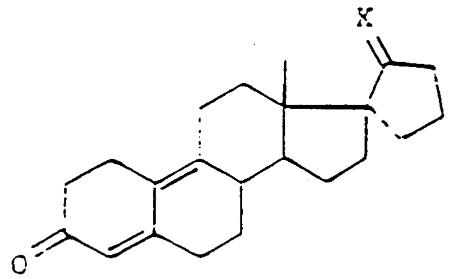

$(I_b)$

corresponding to the products of formula (I) in which the dotted lines in position 9'(10') indicate the presence of a double bond and those in position 11'(12') do not represent a double bond, and which products of formula $(I_b)$ are optionally subjected to a protection reaction in position 3, followed by a gentle hydrolysis with a weak acid then a dehydrogenation in order to obtain the products of formula $(I_c)$:

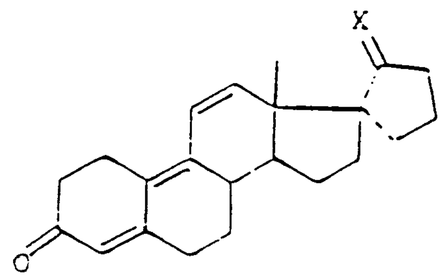

$(I_c)$

corresponding to the products of formula (I) in which the dotted lines in positions 9'(10') and 11'(12') indicate the presence of two additional double bonds in position 9'(10') and 11'(12'),
and if desired, the products of formulae $(I_a)$, $(I_b)$ and $(I_c)$ indicated above and in which =X represents a

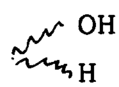

radical are subjected
either to an oxidation reaction in order to obtain the corresponding products of formulae $(I_a)$, $(I_b)$ and $(I_c)$ in which X represents an oxygen atom,
or to an acylation reaction in order to obtain the products in which =X represents a

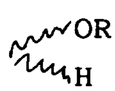

radical in which R represents an acyl radical.

**2.** Process according to claim 1 for the preparation of the products of formula (I') corresponding to the products of general formula (I) as described in claim 1 in which =X represents a

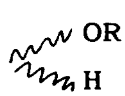

radical, R having the definition indicated in claim 1, characterized in that a product of formula (II'):

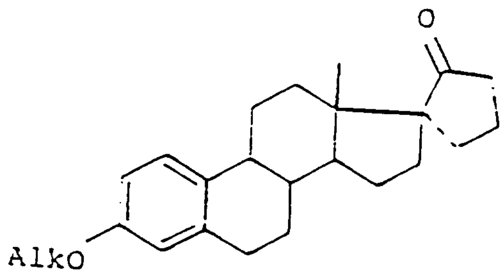

(II')

is subjected to a reduction reaction in order to obtain the products of formula (III):

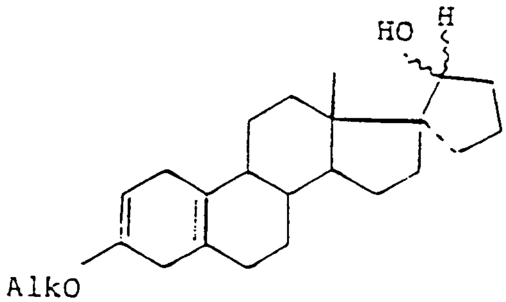

(III)

which products of formula (III) are separated if desired into each of their isomers and the products of formula (III) in the form of epimer mixtures or in the form of pure products are subjected
either to a hydrolysis with a strong acid in order to obtain the products of formula ($I'_a$):

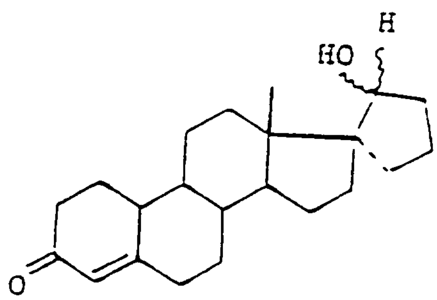

($I'_a$)

which products of formula ($I'_a$) are separated if desired into each of their isomers,
or to a gentle hydrolysis with a weak acid followed by a dehydrogenation in order to obtain the products of formula ($I'_b$):

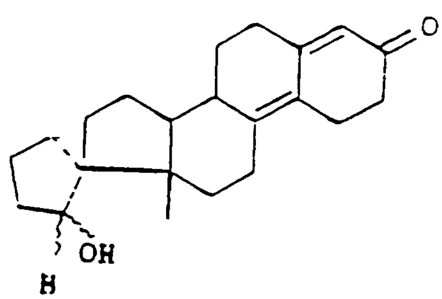

($I'_b$)

which products of formula ($I'_b$) are separated if desired into each of their isomers and which products of formula ($I'_b$) in the form of epimer mixtures or in the form of pure products are optionally subjected to a protection reaction in position 3 followed by a gentle hydrolysis with a weak acid then a dehydrogenation in order to obtain the products of formula ($I'_c$):

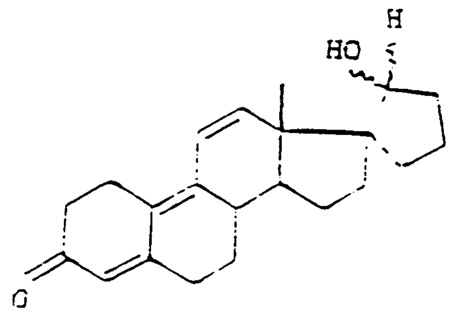

(I'c)

which products of formula (I'$_c$) are separated if desired into each of their isomers and if desired the products of formulae (I'$_a$), (I'$_b$) and (I'$_c$) or if appropriate their isomers are subjected to an acylation reaction in order to obtain the corresponding products in which =X represents a

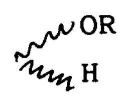

radical in which R represents an acyl radical.

**3.** Process according to claim 1, characterized in that spiro (cyclopentane-1,17'beta-estra-4'9'-diene) 2,3-dione is prepared.

**4.** Preparation process for pharmaceutical compositions, characterized in that at least one of the pharmaceutically acceptable products of formula (I), as defined in claim 1 or 2, is used as active ingredient in a form intended for this use.

**5.** Preparation process for pharmaceutical compositions, characterized in that at least one of the pharmaceutically acceptable products of formula (I), as defined in claim 3, is used as active ingredient in a form intended for this use.

**Patentansprüche**

**Patentansprüche für folgende Vertragsstaaten : AT, BE, CH, DE, DK, FR, GB, IT, LI, LU, NL, SE**

**1.** Produkte der allgemeinen Formel (I)

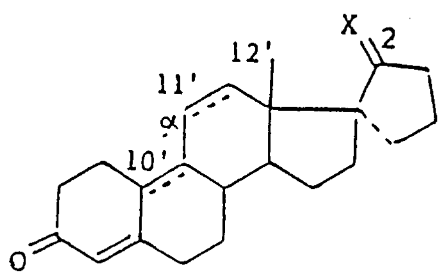

(I)

worin =X für eine Ketonfunktion oder einen Rest

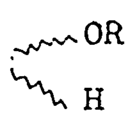

steht, worin R ein Wasserstoffatom oder einen Acylrest mit höchstens 12 Kohlenstoffatomen darstellt und die gewellten Linien anzeigen, daß sich die Substituenten OR und Wasserstoff in $\alpha$- oder $\beta$-

Stellung befinden können, die gestrichelten Linien in den 9'(10')- und 11'(12')-Stellungen die etwaige Anwesenheit einer zweiten Doppelbindung in 9'(10')-Stellung oder von zwei zusätzlichen Doppelbindungen in den 9'(10')- und 11'(12')-Stellungen anzeigen, in Form eines jeden der 2R- oder 2S-Epimeren oder in Form eines Gemisches dieser Epimeren, wobei die gestrichelte Linie in 17-Stellung anzeigt, daß sich die Bindung in $\alpha$-Stellung befindet.

**2.** Produkte der allgemeinen Formel (I), wie in Anspruch 1 definiert, worin X ein Sauerstoffatom bedeutet und die gestrichelte Linie in 11'(12')-Stellung nicht die Anwesenheit einer zweiten Bindung zwischen den sie tragenden Kohlenstoffen anzeigt.

**3.** Spiro-(cyclopentan-1,17'$\beta$-östra-4',9'-dien-2,3'-dion.

**4.** Verfahren zur Herstellung der Produkte der allgemeinen Formel (I), wie in Anspruch 1 definiert, dadurch gekennzeichnet, daß man ein Produkt der Formel (II)

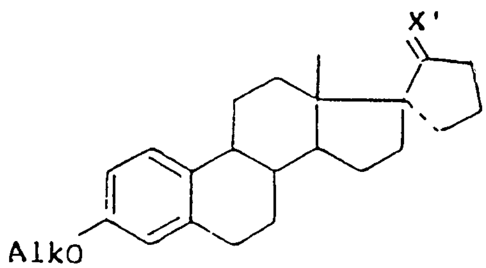

(II)

worin =X' eine gegebenenfalls geschützte Ketonfunktion oder einen Rest

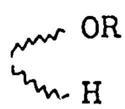

darstellt, wobei R die in Anspruch 1 angegebene Bedeutung besitzt, in Form eines jeden der 2R- oder 2S-Epimeren oder in Form eines Gemisches dieser Epimeren, und wobei Alk einen Alkylrest mit 1 bis 4 Kohlenstoffatomen darstellt,
entweder einer Reduktion und einer anschließenden Hydrolyse mit einer starken Säure unterzieht, um zu den Produkten der Formel ($I_a$)

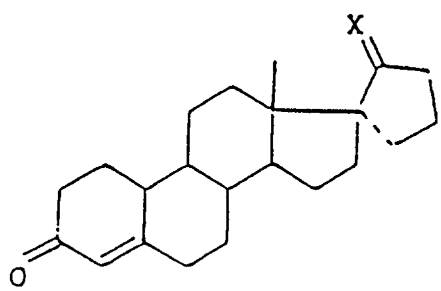

($I_a$)

zu gelangen, worin X die in Anspruch 1 angegebene Bedeutung besitzt, die den Produkten der Formel (I) entsprechen, worin die gestrichelten Linien in 9'(10')- und 11'(12')-Stellung keine zweite Bindung zwischen den sie tragenden Kohlenstoffatomen wiedergeben,
oder einer Reduktion und einer anschließenden milden Hydrolyse mit einer schwachen Säure, danach einer Dehydrierung unterzieht, um zu den Produkten der Formel ($I_b$)

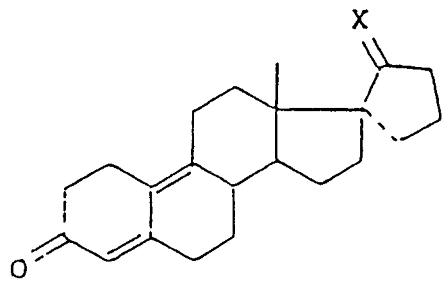

($I_b$)

zu gelangen, die den Produkten der Formel (I) entsprechen, worin die gestrichelten Linien in 9'(10')-Stellung die Anwesenheit einer Doppelbindung anzeigen und diejenigen in 11'(12')-Stellung keine Doppelbindung wiedergeben, welche Produkte der Formel ($I_b$) man gegebenenfalls einer Reaktion für den Schutz in 3-Stellung unterzieht, woran sich eine milde Hydrolyse mit einer schwachen Säure und danach eine Dehydrierung anschließt, um zu den Produkten der Formel ($I_c$)

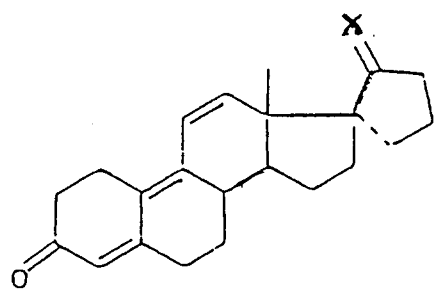

($I_c$)

zu gelangen, die den Produkten der Formel (I) entsprechen, worin die gestrichelten Linien in 9'(10')- und 11'(12')-Stellung die Anwesenheit von zwei zusätzlichen Doppelbindungen in 9'(10')-und 11'(12')-Stellung anzeigen, und gewünschtenfalls die vorstehend angegebenen Produkte der Formeln ($I_a$), ($I_b$) und ($I_c$) , worin =X für einen Rest

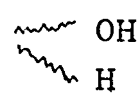

steht, entweder einer Oxidationsreaktion unterzieht, um zu den entsprechenden Produkten der Formeln ($I_a$), ($I_b$) und ($I_c$) zu gelangen, worin X für ein Sauerstoffatom steht,
oder einer Acylierungsreaktion unterzieht, um zu den Produkten zu gelangen, worin =X für einen Rest

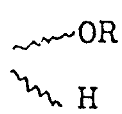

steht, in dem R einen Acylrest bedeutet.

5. Verfahren gemäß Anspruch 4 zur Herstellung der Produkte der Formel (I'), die den Produkten der allgemeinen Formel (I), wie in Anspruch 1 definiert, entsprechen, worin =X für einen Rest

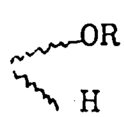

steht, wobei R die in Anspruch 1 angegebene Bedeutung besitzt,

dadurch gekennzeichnet, daß man ein Produkt der Formel (II')

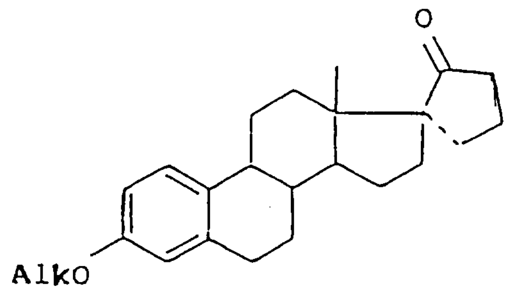

(II')

einer Reduktionsreaktion unterzieht, um zu den Produkten der Formel (III)

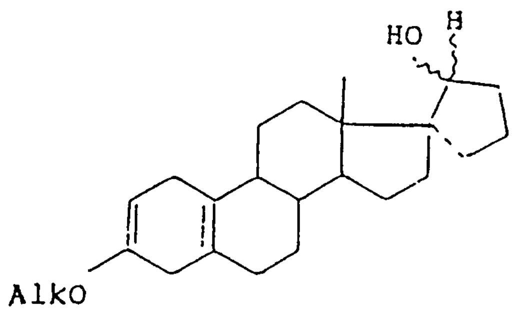

(III)

zu gelangen, welche Produkte der Formel (III) man gewünschtenfalls in ein jedes ihrer Isomeren auftrennt und die Produkte der Formel (III) in Form des Epimerengemisches oder in Form der reinen Produkte
entweder einer Hydrolyse mit einer starken Säure unterzieht, um zu den Produkten der Formel ($I'_a$)

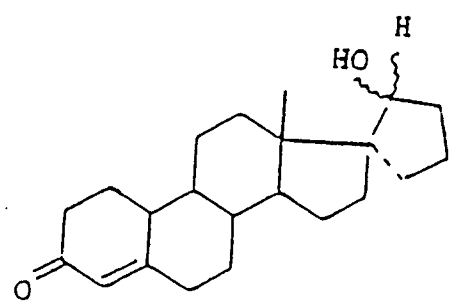

($I'_a$)

zu gelangen, welche Produkte der Formel ($I'_a$) man gewünschtenfalls in ein jedes ihrer Isomeren auftrennt,
oder einer milden Hydrolyse mit einer schwachen Säure und einer sich anschließenden Dehydrierung unterzieht, um zu den Produkten der Formel ($I'_b$)

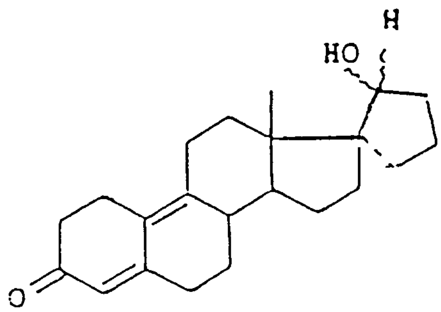

(I'$_b$)

zu gelangen, welche Produkte der Formel (I'$_b$) man gewünschtenfalls in ein jedes ihrer Isomeren auftrennt, und die Produkte der Formel (I'$_b$) in Form des Epimerengemisches oder in Form der reinen Produkte man gegebenenfalls einer Reaktion für den Schutz in 3-Stellung unterzieht, woran sich eine milde Hydrolyse mit einer schwachen Säure und nachfolgend eine Dehydrierung anschließt, um zu den Produkten der Formel (I'$_c$)

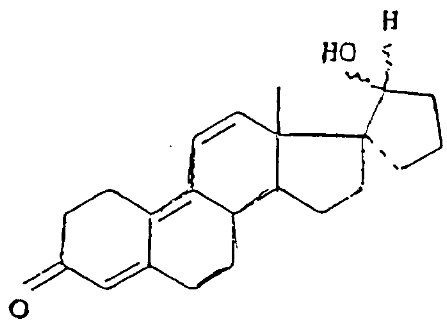

(I'$_c$)

zu gelangen, welche Produkte der Formel (I'$_c$) man gewünschtenfalls in jedes ihrer Isomeren auftrennt und gewünschtenfalls die Produkte der Formeln (I'$_a$), (I'$_b$) und (I'$_c$) oder gegebenenfalls ihre Isomeren einer Acylierungsreaktion unterzieht, um zu den entsprechenden Produkten zu gelangen,
worin =X für einen Rest

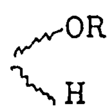

steht, worin R einen Acylrest bedeutet.

6. Als Arzneimittel die Produkte der Formel (I), wie in einem der Ansprüche 1 oder 2 definiert.

7. Als Arzneimittel das in Anspruch 3 definierte Produkt.

8. Pharmazeutische Zusammensetzungen, enthaltend als Wirkstoff zumindest ein Arzneimittel, wie in einem der Ansprüche 6 oder 7 definiert.

9. Als neue industrielle Produkte die Produkte der Formeln (II) und (III)

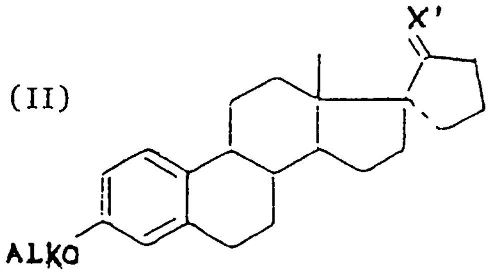

(II)

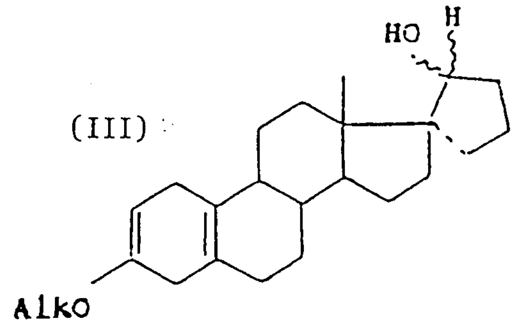

(III)

worin =X' eine gegebenenfalls geschützte Ketonfunktion oder einen Rest

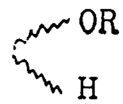

wiedergibt, wobei R die in Anspruch 1 angegebene Bedeutung besitzt, in Form eines jeden der 2R- oder 2S-Epimeren oder in Form eines Gemisches dieser Epimeren, und Alk einen Alkylrest mit 1 bis 4 Kohlenstoffatomen darstellt.

**Patentansprüche für folgenden Vertragsstaat : ES**

1. Verfahren zur Herstellung der Produkte der allgemeinen Formel (I)

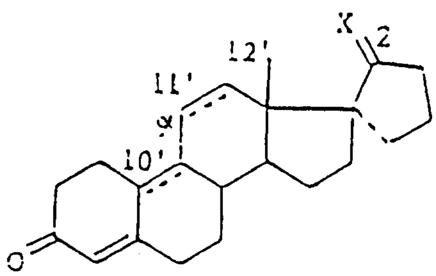

(I)

worin =X für eine Ketonfunktion oder einen Rest

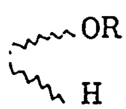

steht, worin R ein Wasserstoffatom oder einen Acylrest mit höchstens 12 Kohlenstoffatomen darstellt und die gewellten Linien anzeigen, daß sich die Substituenten OR und Wasserstoff in α- oder β-Stellung befinden können, die gestrichelten Linien in den 9'(10')- und 11'(12')-Stellungen die etwaige Anwesenheit einer zweiten Doppelbindung in 9'(10')-Stellung oder von zwei zusätzlichen Doppelbindungen in den 9'(10')- und 11'(12')-Stellungen anzeigen, in Form eines jeden der 2R- oder 2S-Epimeren oder in Form eines Gemisches dieser Epimeren, wobei die gestrichelte Linie in 17-Stellung anzeigt, daß sich die Bindung in α-Stellung befindet, dadurch gekennzeichnet, daß man ein Produkt der Formel (II)

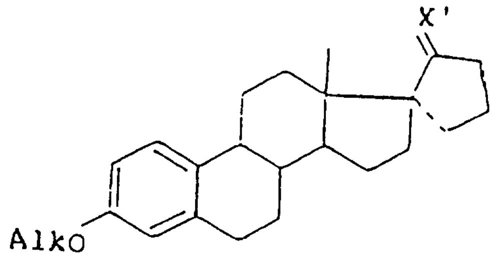

(II)

worin =X' eine gegebenenfalls geschützte Ketonfunktion oder einen Rest

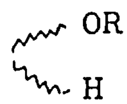

darstellt, wobei R die vorstehend angegebene Bedeutung besitzt, in Form eines jeden der 2R- oder 2S-Epimeren oder in Form eines Gemisches dieser Epimeren, und wobei Alk einen Alkylrest mit 1 bis 4 Kohlenstoffatomen darstellt,
entweder einer Reduktion und einer anschließenden Hydrolyse mit einer starken Säure unterzieht, um zu den Produkten der Formel ($I_a$)

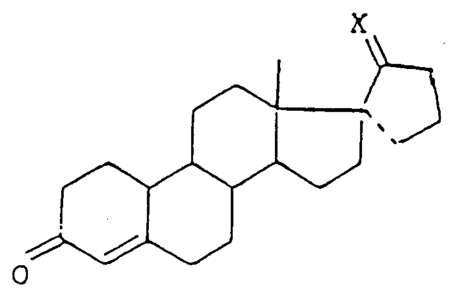

($I_a$)

zu gelangen, worin X die vorstehend angegebene Bedeutung besitzt, die den Produkten der Formel (I) entsprechen, worin die gestrichelten Linien in 9'(10')- und 11'(12')-Stellung keine zweite Bindung zwischen den sie tragenden Kohlenstoffatomen wiedergeben,
oder einer Reduktion und einer anschließenden milden Hydrolyse mit einer schwachen Säure, danach einer Dehydrierung unterzieht, um zu den Produkten der Formel ($I_b$)

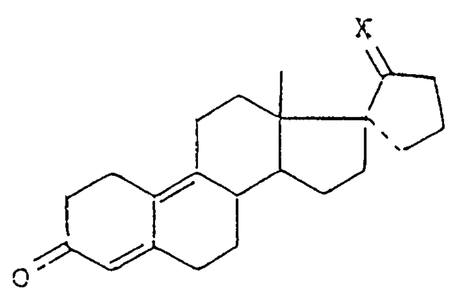

($I_b$)

zu gelangen, die den Produkten der Formel (I) entsprechen, worin die gestrichelten Linien in 9'(10')-Stellung die Anwesenheit einer Doppelbindung anzeigen und diejenigen in 11'(12')-Stellung keine Doppelbindung wiedergeben, welche Produkte der Formel ($I_b$) man gegebenenfalls einer Reaktion für den Schutz in 3-Stellung unterzieht, woran sich eine milde Hydrolyse mit einer schwachen Säure und danach eine Dehydrierung anschließt, um zu den Produkten der Formel ($I_c$)

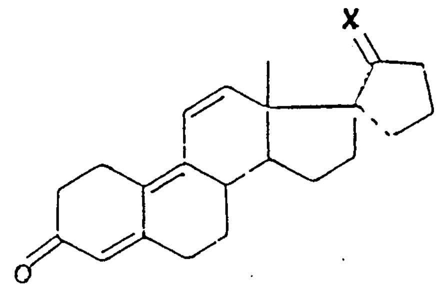

$(I_c)$

zu gelangen, die den Produkten der Formel (I) entsprechen, worin die gestrichelten Linien in 9'(10')- und 11'(12')-Stellung die Anwesenheit von zwei zusätzlichen Doppelbindungen in 9'(10')-und 11'(12')-Stellung anzeigen, und gewünschtenfalls die vorstehend angegebenen Produkte der Formeln $(I_a)$, $(I_b)$ und $(I_c)$ , worin =X für einen Rest

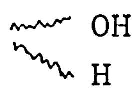

steht, entweder einer Oxidationsreaktion unterzieht, um zu den entsprechenden Produkten der Formeln $(I_a)$, $(I_b)$ und $(I_c)$ zu gelangen, worin X für ein Sauerstoffatom steht,
oder einer Acylierungsreaktion unterzieht, um zu den Produkten zu gelangen, worin =X für einen Rest

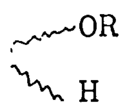

steht, in dem R einen Acylrest bedeutet.

**2.** Verfahren gemäß Anspruch 1 zur Herstellung der Produkte der Formel (I'), die den Produkten der allgemeinen Formel (I), wie in Anspruch 1 definiert, entsprechen, worin =X für einen Rest

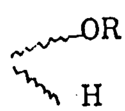

steht, wobei R die in Anspruch 1 angegebene Bedeutung besitzt,
dadurch gekennzeichnet, daß man ein Produkt der Formel (II')

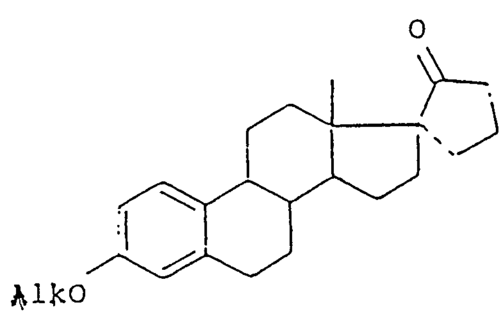

(II')

einer Reduktionsreaktion unterzieht, um zu den Produkten der Formel (III)

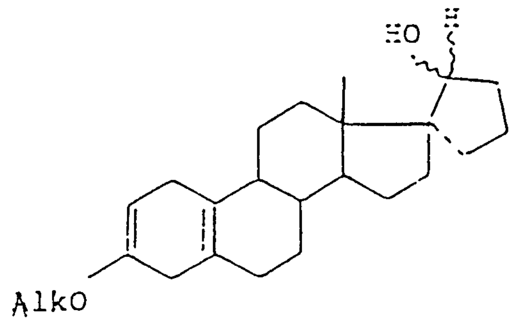

(III)

zu gelangen, welche Produkte der Formel (III) man gewünschtenfalls in ein jedes ihrer Isomeren auftrennt und die Produkte der Formel (III) in Form des Epimerengemisches oder in Form der reinen Produkte

entweder einer Hydrolyse mit einer starken Säure unterzieht, um zu den Produkten der Formel ($I'_a$)

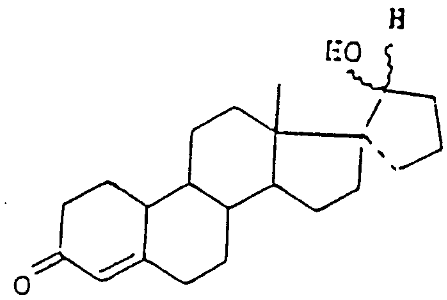

($I'_a$)

zu gelangen, welche Produkte der Formel ($I'_a$) man gewünschtenfalls in ein jedes ihrer Isomeren auftrennt,

oder einer milden Hydrolyse mit einer schwachen Säure und einer sich anschließenden Dehydrierung unterzieht, um zu den Produkten der Formel ($I'_b$)

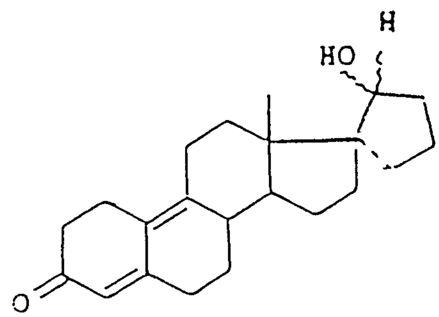

($I'_b$)

zu gelangen, welche Produkte der Formel ($I'_b$) man gewünschtenfalls in ein jedes ihrer Isomeren auftrennt, und die Produkte der Formel ($I'_b$) in Form des Epimerengemisches oder in Form der reinen Produkte man gegebenenfalls einer Reaktion für den Schutz in 3-Stellung unterzieht, woran sich eine milde Hydrolyse mit einer schwachen Säure und nachfolgend eine Dehydrierung anschließt, um zu den Produkten der Formel ($I'_c$)

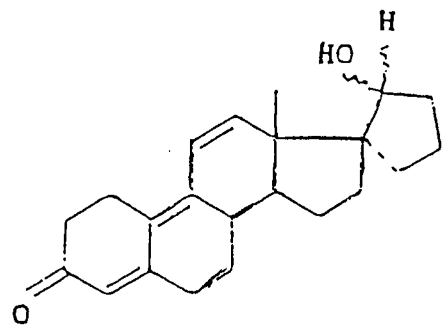

(I'$_c$)

zu gelangen, welche Produkte der Formel (I'$_c$) man gewünschtenfalls in jedes ihrer Isomeren auftrennt und gewünschtenfalls die Produkte der Formeln (I'$_a$), (I'$_b$) und (I'$_c$) oder gegebenenfalls ihre Isomeren einer Acylierungsreaktion unterzieht, um zu den entsprechenden Produkten zu gelangen, worin =X für einen Rest

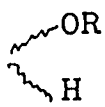

steht, worin R einen Acylrest bedeutet.

**Patentansprüche für folgenden Vertragsstaat : GR**

**1.** Verfahren zur Herstellung der Produkte der allgemeinen Formel (I)

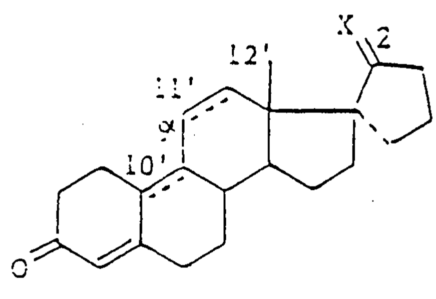

(I)

worin =X für eine Ketonfunktion oder einen Rest

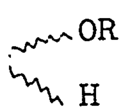

steht, worin R ein Wasserstoffatom oder einen Acylrest mit höchstens 12 Kohlenstoffatomen darstellt und die gewellten Linien anzeigen, daß sich die Substituenten OR und Wasserstoff in α- oder β-Stellung befinden können, die gestrichelten Linien in den 9'(10')- und 11'(12')-Stellungen die etwaige Anwesenheit einer zweiten Doppelbindung in 9'(10')-Stellung oder von zwei zusätzlichen Doppelbindungen in den 9'(10')-und 11'(12')Stellungen anzeigen, in Form eines jeden der 2R- oder 2S-Epimeren oder in Form eines Gemisches dieser Epimeren, wobei die gestrichelte Linie in 17-Stellung anzeigt, daß sich die Bindung in α-Stellung befindet, dadurch gekennzeichnet, daß man ein Produkt der Formel (II)

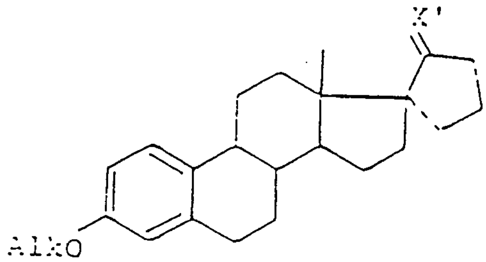

(II)

worin =X' eine gegebenenfalls geschützte Ketonfunktion oder einen Rest

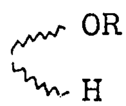

darstellt, wobei R die vorstehend angegebene Bedeutung besitzt, in Form eines jeden der 2R- oder 2S-Epimeren oder in Form eines Gemisches dieser Epimeren, und wobei Alk einen Alkylrest mit 1 bis 4 Kohlenstoffatomen darstellt,
entweder einer Reduktion und einer anschließenden Hydrolyse mit einer starken Säure unterzieht, um zu den Produkten der Formel ($I_a$)

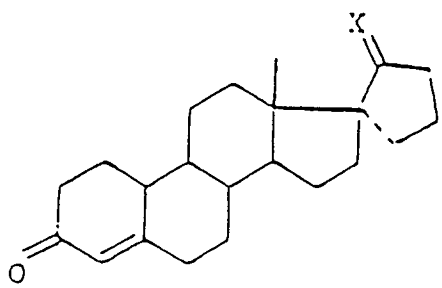

($I_a$)

zu gelangen, worin X die vorstehend angegebene Bedeutung besitzt, die den Produkten der Formel (I) entsprechen, worin die gestrichelten Linien in 9'(10')- und 11'(12')-Stellung keine zweite Bindung zwischen den sie tragenden Kohlenstoffatomen wiedergeben,
oder einer Reduktion und einer anschließenden milden Hydrolyse mit einer schwachen Säure, danach einer Dehydrierung unterzieht, um zu den Produkten der Formel ($I_b$)

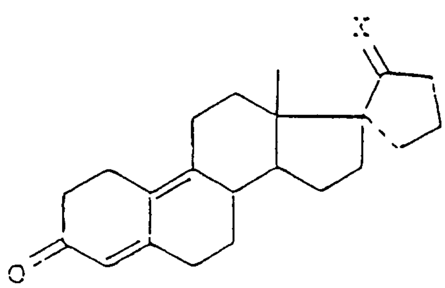

($I_b$)

zu gelangen, die den Produkten der Formel (I) entsprechen, worin die gestrichelten Linien in 9'(10')-Stellung die Anwesenheit einer Doppelbindung anzeigen und diejenigen in 11'(12')-Stellung keine Doppelbindung wiedergeben, welche Produkte der Formel ($I_b$) man gegebenenfalls einer Reaktion für den Schutz in 3-Stellung unterzieht, woran sich eine milde Hydrolyse mit einer schwachen Säure und danach eine Dehydrierung anschließt, um zu den Produkten der Formel ($I_c$)

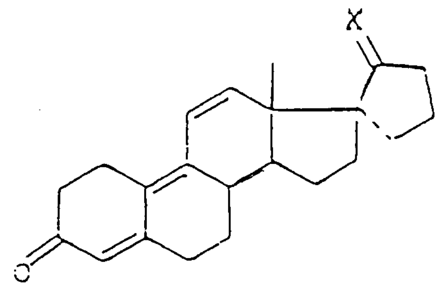

$(I_c)$

zu gelangen, die den Produkten der Formel (I) entsprechen, worin die gestrichelten Linien in 9'(10')- und 11'(12')-Stellung die Anwesenheit von zwei zusätzlichen Doppelbindungen in 9'(10')-und 11'(12')-Stellung anzeigen, und gewünschtenfalls die vorstehend angegebenen Produkte der Formeln $(I_a)$, $(I_b)$ und $(I_c)$ , worin =X für einen Rest

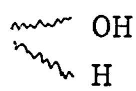

steht, entweder einer Oxidationsreaktion unterzieht, um zu den entsprechenden Produkten der Formeln $(I_a)$, $(I_b)$ und $(I_c)$ zu gelangen, worin X für ein Sauerstoffatom steht,
oder einer Acylierungsreaktion unterzieht, um zu den Produkten zu gelangen, worin =X für einen Rest

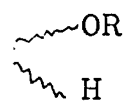

steht, in dem R einen Acylrest bedeutet.

**2.** Verfahren gemäß Anspruch 1 zur Herstellung der Produkte der Formel (I'), die den Produkten der allgemeinen Formel (I), wie in Anspruch 1 definiert, entsprechen, worin =X für einen Rest

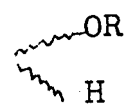

steht, wobei R die in Anspruch 1 angegebene Bedeutung besitzt,
dadurch gekennzeichnet, daß man ein Produkt der Formel (II')

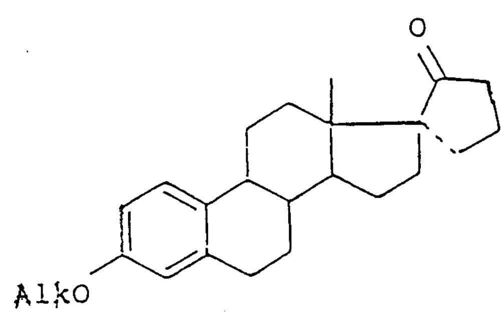

(II')

einer Reduktionsreaktion unterzieht, um zu den Produkten der Formel (III)

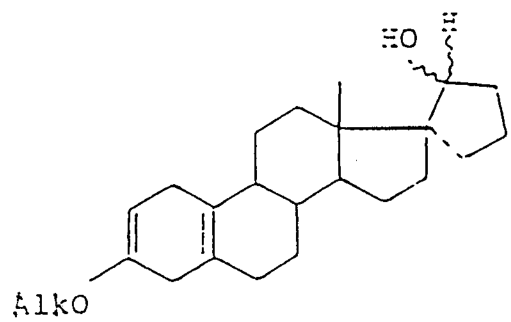

(III)

zu gelangen, welche Produkte der Formel (III) man gewünschtenfalls in ein jedes ihrer Isomeren auftrennt und die Produkte der Formel (III) in Form des Epimerengemisches oder in Form der reinen Produkte

entweder einer Hydrolyse mit einer starken Säure unterzieht, um zu den Produkten der Formel ($I'_a$)

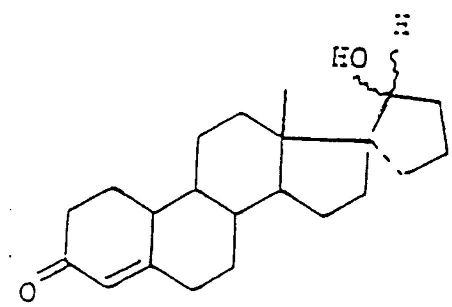

($I'_a$)

zu gelangen, welche Produkte der Formel ($I'_a$) man gewünschtenfalls in ein jedes ihrer Isomeren auftrennt,

oder einer milden Hydrolyse mit einer schwachen Säure und einer sich anschließenden Dehydrierung unterzieht, um zu den Produkten der Formel ($I'_b$)

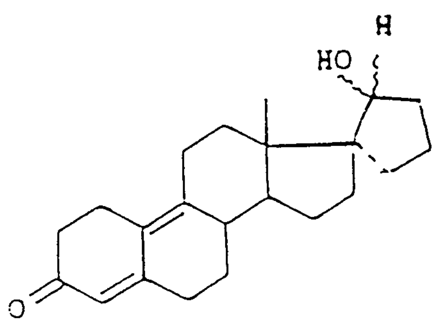

($I'_b$)

zu gelangen, welche Produkte der Formel ($I'_b$) man gewünschtenfalls in ein jedes ihrer Isomeren auftrennt, und die Produkte der Formel ($I'_b$) in Form des Epimerengemisches oder in Form der reinen Produkte man gegebenenfalls einer Reaktion für den Schutz in 3-Stellung unterzieht, woran sich eine milde Hydrolyse mit einer schwachen Säure und nachfolgend eine Dehydrierung anschließt, um zu den Produkten der Formel ($I'_c$)

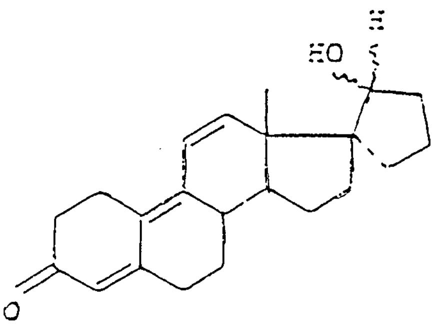

(I'$_c$)

zu gelangen, welche Produkte der Formel (I'$_c$) man gewünschtenfalls in jedes ihrer Isomeren auftrennt und gewünschtenfalls die Produkte der Formeln (I'$_a$), (I'$_b$) und (I'$_c$) oder gegebenenfalls ihre Isomeren einer Acylierungsreaktion unterzieht, um zu den entsprechenden Produkten zu gelangen, worin =X für einen Rest

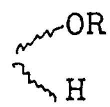

steht, worin R einen Acylrest bedeutet.

**3.** Verfahren gemäß Anspruch 1, dadurch gekennzeichnet, daß man Spiro-(cyclopentan-1,17'-$\beta$-östra-4',9'-dien)-2,3'-dion herstellt.

**4.** Verfahren zur Herstellung von pharmazeutischen Zusammensetzungen, dadurch gekennzeichnet, daß man als Wirkstoff zumindest eines der pharmazeutisch annehmbaren Produkte der Formel (I), wie in Anspruch 1 oder 2 definiert, in eine für diese Verwendung bestimmte Form überführt.

**5.** Verfahren zur Herstellung von pharmazeutischen Zusammensetzungen, dadurch gekennzeichnet, daß man als Wirkstoff zumindest eines der pharmazeutisch verträglichen Produkte der Formel (I), wie in Anspruch 3 definiert, in eine für diese Verwendung bestimmte Form überführt.